# EUROPÄISCHE PATENTSCHRIFT

(11) **EP 1 667 770 B1**
(45) Veröffentlichungstag und Bekanntmachung des Hinweises auf die Patenterteilung: **11.03.2009**
(21) Anmeldenummer: 04761031.6
(22) Anmeldetag: 09.09.2004
(51) Int. Cl.: A61P 25/28, A61P 25/14, A61P 25/16, A61P 3/10, A61P 25/08, A61P 9/10, A61P 25/00, A61K 31/55, C07D 491/10

(54) **VERFAHREN ZUR HERSTELLUNG VON DERIVATEN DES 4A,5,9,10,11,12-HEXAHYDROBENZOFURO [3A,3,2][ 2 ]- BENZAZEPINS**
METHOD FOR THE PRODUCTION OF DERIVATIVES OF 4A,5,9,10,11,12-HEXAHYDROBENZOFURO [3A,3,2][ 2]- BENZAZEPINE
PROCEDE POUR PREPARER DES DERIVES DE 4A,5,9,10,11,12-HEXAHYDROBENZOFURO [3A,3,2][ 2]- BENZAZEPINE

(30) Priorität: 29.09.2003 AT 15382003; 12.07.2004 AT 11742004
(43) Veröffentlichungstag der Anmeldung: 14.06.2006
(73) Patentinhaber: Sanochemia Pharmazeutika Aktiengesellschaft, 1090 Wien (AT)
(72) Erfinder: CZOLLNER, Laszlo, A-2490 Ebenfurth (AT); KÄLZ, Beate, A-7035 Steinbrunn (AT); WELZIG, Stefan, A-1030 Wien (AT); FRANTSITS, Werner, J., A-1130 Wien (AT); JORDIS, Ulrich, A-1180 Wien (AT); FRÖHLICH, Johannes, A-2392 Dornbach/Wienerwald (AT)
(74) Vertreter: Dungler, Karin
(86) Internationale Anmeldenummer: PCT/AT2004/000309
(87) Internationale Veröffentlichungsnummer: WO 2005/030333

(56) Entgegenhaltungen:
- WO-A-00/32185
- WO-A-00/32199
- WO-A-01/74820
- WO-A-96/12692
- WO-A-97/03987
- WO-A-97/40049
- FR-A- 2 826 005
- NL-A- 8 800 350
- BHANDARKAR J G ET AL: "STRUCTURE AND BIOSYNTHESIS OF CHLIDANTHINE" JOURNAL OF THE CHEMICAL SOCIETY, SECTION C: ORGANIC CHEMISTRY, CHEMICAL SOCIETY. LETCHWORTH, GB, Nr. 9, 1970, Seiten 1224-1227, XP008043788 ISSN: 0022-4952
- BARTOLUCCI C ET AL: "THREE-DIMENSIONAL STRUCTURE OF A COMPLEX OF GALANTHAMINE (NIVALIN) WITH ACETYLCHOLINESTERASE FROM TORPEDO CALIFORNICA: IMPLICATIONS FOR THE DESIGN OF NEW ANTI-ALZHEIMER DRUGS" PROTEINS: STRUCTURE, FUNCTION AND GENETICS, ALAN R. LISS, US, Bd. 42, Nr. 2, 2001, Seiten 182-191, XP008043735 ISSN: 0887-3585
- PILGER C ET AL: "ACCURATE PREDICTION OF THE BOUND CONFORMATION OF GALANTHAMINE IN THE ACTIVE SITE OF TORPEDO CALIFORNICA ACETYLCHOLINESTERASE USING MOLECULAR DOCKING" JOURNAL OF MOLECULAR GRAPHICS AND MODELLING, ELSEVIER SCIENCE, NEW YORK, NY, US, Bd. 19, Nr. 3/4, 2001, Seiten 288-296, XP008043734 ISSN: 1093-3263
- RECANATINI M ET AL: "ACETYLCHOLINESTERASE INHIBITORS IN THE CONTEXT OF THERAPEUTIC STRATEGIES TO COMBAT ALZHEIMER'S DISEASE" EXPERT OPINION ON THERAPEUTIC PATENTS, ASHLEY PUBLICATIONS, GB, Bd. 12, Nr. 12, 2002, Seiten 1853-1865, XP008043793 ISSN: 1354-3776
- BASTIDA J ET AL: "ALKALOIDS FROM NARCISSUS LEONENSIS" PHYTOCHEMISTRY, PERGAMON PRESS, GB, Bd. 34, Nr. 6, 1993, Seiten 1656-1658, XP001009775 ISSN: 0031-9422
- VLAHOV R ET AL: "SYNTHESIS OF GALANTHAMINE AND RELATED ALKALOIDS - NEW APPROACHES. I" TETRAHEDRON, ELSEVIER SCIENCE PUBLISHERS, AMSTERDAM, NL, Bd. 45, Nr. 11, 1989, Seiten 3329-3345, XP000562833 ISSN: 0040-4020
- MUTSCHLER ET AL.: "Arzneimittelwirkungen" 2001, WVG , STUTTGART , XP002306319 Seite 195, Spalte 2, Absatz 1
- LI H-Y ET AL: "ALKALOIDS OF LYCORIS GUANGXIENSIS" PLANTA MEDICA, THIEME, STUTTGART, DE, Bd. 53, Nr. 3, 1987, Seiten 259-261, XP008043740 ISSN: 0032-0943

## Beschreibung

Die Erfindung betrifft Verfahren zur Herstellung von Derivaten des 4a,5,9,10,11,12-Hexahydro-benzofuro [3a,3,2] [2] benzazepin.

Zu dem eingangs genannten Verbindungstyp zählen unter anderem auch Galanthaminderivate.

Galanthamin ist ein tetracyclisches Alkaloid, welches zur Gruppe der reversibel wirkenden Cholinesterasehemmstoffe gehört und auch als Wirkstoff in der Alzheimertherapie - siehe Neurologist 9, 235, 2003; Clinical Geriatrics 9(11), 55, 2001 - Anwendung findet. Es ist weiters aus der Literatur bekannt, dass Strukturanaloga des natürlich vorkommenden Galanthamin unterschiedliche chemische Eigenschaften aufweisen - siehe Proc. Chem. Soc. 357, 1964. Somit führt eine Veränderung der räumlichen Anordnung von Substituenten an einem asymmetrischen Kohlenstoffatom zu einer signifikanten Änderung der pharmakologischen Eigenschaften - siehe Farmakol. Alkaloidov Serdech. Glikozidov 96, 1971, Russ. Hinsichtlich pharmakologische Eigenschaften ist insbesondere die räumliche Anordnung am Kohlenstoff 6 des Galanthamin-Grundkörpers maßgeblich.

Trotzdem eine Vielzahl von Verfahren zur Herstellung von Galanthamin bekannt ist, war es bisher nicht möglich, optisch aktive Derivate der vorgenannten 6-Epianaloga von natürlich vorkommenden oder synthetischem Galanthamin herzustellen, weil die zur Synthese benötigten optisch aktiven Zwischenprodukte, das sind 11-Demethyl-6-epigalanthamine, nicht zugänglich waren. Chirale Trennungen des 11-Demethyl-Galanthamin und 11-Demethyl-Bromgalanthamin sind beispielsweise in der WO-A-96/12692, WO-A-97/40049 und WO-A-01/74820 beschrieben. Das (-)-11-Demethyl-Galanthamin kann aus Pflanzenextrakt - sieh Nat. Prod. Sci. 4, 148, 1998 - oder auf synthetischem Weg - siehe US-A-5958903, WO-A-03/080623, WO-A-97/03987) - aus (-)-Galanthamin erhalten werden. Lediglich aus Phytochemistry 34, 1656, 1993 ist es bekannt, im Milligrammbereich ein racemisches Gemisch eines 1-Brom-Derivates von 11-Demethyl-epigalanthamin aus einem Pflanzenextrakt zu gewinnen.

Der Erfindung liegt daher die Aufgabe zugrunde, einen Beitrag für die Bereitstellung von (+) und auch (-) -11-Demethyl-6-epigalanthamin zu liefern, welcher auch eine effiziente Verwendung im Industriemaßstab ermöglichen soll.

Erfindungsgemäß werden Verfahren zur Herstellung von Derivaten des 4a,5,9,10,11,12-Hexahydro-benzofuro[3a,3,2][2] benzazepin mit der allgemeinen Formel Ia bzw. Ib 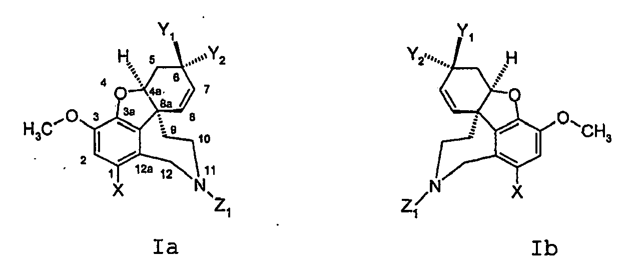 sowie deren Salze vorgeschlagen, wobei
- Ia optisch aktive (-) Derivate des Galanthamin und Ib optisch aktive (+) -Derivate des Galanthamin sind, die in zu einander spiegelbildlich räumlicher Anordnung vorliegen, und worin
- Y₁ und Y₂ wechselweise H oder OH,
- X = H oder Br sind und
- Z₁ = eine Gruppe mit folgender Formeldarstellung 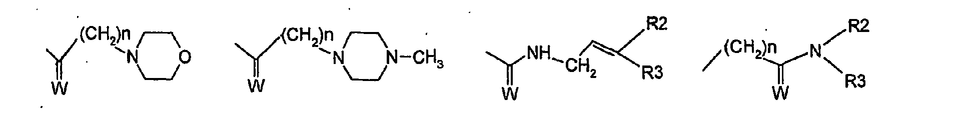 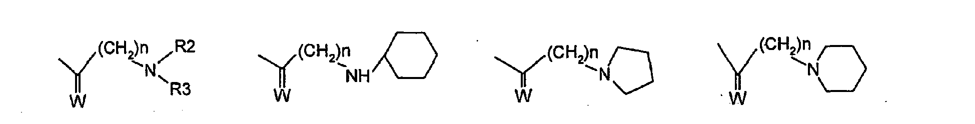 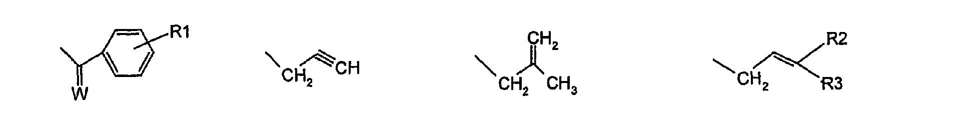 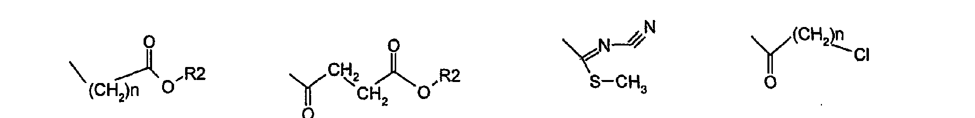 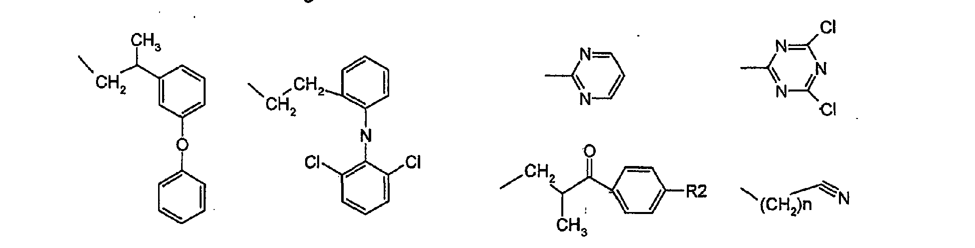
ist, worin
- R₁ = H, Cl, Br, I, F, OH, geradkettiges oder verzweigtes (C₁-C₆) alkyl, geradkettiges oder verzweigtes (C₁-C₆) alkyloxy, NO₂, NR₂R₃,
- R₂ = R₃ = H, geradkettiges oder verzweigtes (C₁-C₆) alkyl
- W = H, O, S
- n = 0, 1-6 sind,

Erfindungsgemäß erfolgt die Herstellung der Verbindungen Ia und Ib durch Umwandlung von natürlichem und auch synthetischem 11-Demethyl-Galanthamin in die entsprechenden 5-Epianaloga. Durch Behandlung mit verdünnter Säure ist dieses Verfahren auch für die Herstellung von optisch aktiven Derivaten des 11-Demethyl-6-epigalanthamin geeignet, weil während der Herstellung nur die Konfiguration in der Position 6 geändert wird, wogegen die beiden anderen Asymmetriezentren 4a und 8a unverändert bleiben.

Ausgehend von diesen optisch aktiven Ausgangsmaterialien stellt die Erfindung ein effizientes und industriell anwendbares Verfahren zur Herstellung von optisch aktiven Derivaten des (-)-Epi-galanthamin und auch des optisch aktiven (+)-Epigalanthamin zu Verfügung. Durch Anwendung der Erfindung können nicht nur die in der Natur vorkommenden (-)-Derivate, sondern auch die in der Natur nicht vorkommenden (+)- Derivate des 4a,5,9,10,11,12-Hexahydrobenzofuro[3a,3,2][2] benzazepin auf synthetischem Weg hergestellt werden.

Das erfindungsgemäße Verfahren hat den Vorteil, dass die Konfigurationsumwandlungen sowohl bei den natürlichen als auch bei den in der Natur nicht vorkommenden Derivaten mit den optisch aktiven Analogen des Galanthamin und nicht mit den Analogen des 6-EpiGalanthamins durchgeführt werden. Durch diese Methode können nach einmaliger Racemattrennung alle 4 Derivate, nämlich N-Demethyl-Analoge des (-)-Galanthamin, des (+)-Galanthamin sowie (-)-6-Epigalanthamin und (+)-6-Epigalanthamin hergestellt werden.

| Beispiel Nr | Struktur | Stereo | Acetyl-cholinesterase IC-50 (µM) | Butyryl-cholinesterase IC-50(µM) | Name |
|---|---|---|---|---|---|
| 1 | 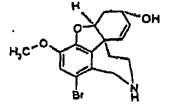 | (-) epi | >100 | >100 | (4aS,6S,6S)-1-Bromo-4a, 5,9,10,11,12-hexahydro-3-methoxy-6H-[1] benzofuro[3a,3,2-ef][2]benzazepin-6-ol, |
| 2 | 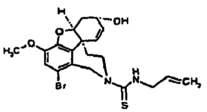 | (-)epi | 51 | >100 | (4aS,6S,8aS)-1-Bromo-6-hydroxy-3- methoxy-4a,5,9,10-tetrahydro-6H-[1]benzofuro[3a,3,2-ef][2]benzazepin-11(12H)-thiocarbonic acid allylamide |
| 3 | 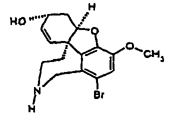 | (+) epi | > 100 | > 100 | (4aR, 6R,8R)-1-Bromo-4a,5,9,10,11,12-hexahydro-3-methoxy-6H-[1]benzofuro[3a,3,2-ef][2]benzazepin-6-ol, |
| 4 | 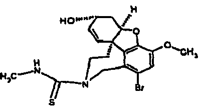 | (+) epi | > 100 | > 100 | (4aR,6R,8aR)-1-Bromo-4a,5,9,10-tetrahydro-6-hydroxy-3-methoxy-6H-[1]benzofuro-[3a,3,2-ef][2]benzazepin-11(12H)-thiocarbonic acid methylamide |
| 5 | 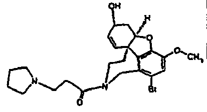 | (+) | >100 | 66 | 1-[(4aR,6S,8aR)-6-Hydroxy-3-methoxy-5.6,9,10-tetrahydro-4aH-[1]benzofuro(3a,3,2-ef][2]benzazepin-11(12H)-yl]-3-(1-pyrrolidyl)propan-1-on |
| 6 | 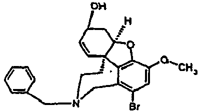 | (+) | >100 | 19 | (4aR,6S,8aR)-11-Benzyl-1-bromo-4a,5,9,10,11,12-Hexahydro-3-methoxy-6H-[1]benzofuro[3a,3,2-ef][2]benzazepin-6-ol |
| 7 | 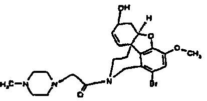 | (+) | 69 | > 100 | 1-[(4aR,6S,8aR)-1-Bromo-6-hydroxy-3-methoxy-5,6,9,10-tetrahydro-4aH-[1]benzofuro[3a,3,2-ef][2]benzazepin-11(12H)-yl]-2-(4-methylpiperazinyl)ethan-1-on |
| 8 | 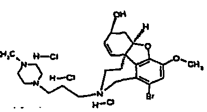 | (+) | > 100 | 31 | (4aR,6R,86aR)11-(3-(4-methylpiperazine)-1-yl-propyl)-3-methoxy-5,6,9,10,11,12-hexahydro-4aH-[1]benzofuro[3a,3,2-ef][2]benzazepin-6-ol, Trihydrochloride |
| 9 | 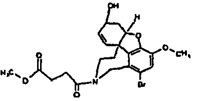 | (+) | >100 | >100 | - Methyl-4-((4aR,65,8aR)-1-Bromo-4a,5,9,10,11,12-hexahydro-6-hydroxy-3-methoxy-6H-benzofuro[3a,3,2-ef][2]benzazepin-11-yl)gamma-oxo-butyrate |
| 10 | 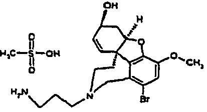 | (+) | >100 | >100 | (4aR, 6S,8aR)-11-(4-aminopropyl)-3-methoxy-5,6,9,10,11,12-hexahydro-4aH-[1]benzofuro[3a,3,2-ef][2]benzazepin-6-ol, Methansulfonate |
| 11 | 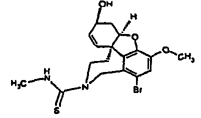 | (+) | > 100 | >100 | (4aR,6S,8aR)-1-Bromo-4a,5,9,10-tetrahydro-6-hydroxy-3-methoxy-6H-[1]benzofuro-[3a,3,2-ef][2]benzazepine-11(12H)-thiocarbonic acid methylamide |
| 12 | 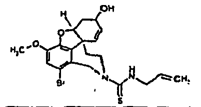 | (-) | >100 | 11 | (4aS,6R,8aS)-1-Bromo-6-hydroxy-3-methoxy-4a,5,9,10-tetrahydro-6H-[1]benzofuro[3a,3,2-ef][2]benzazepin-11(12H)-thiocarbonic acid allylamide |
| 13 | 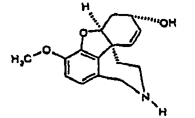 | (-) epi | 15 | 0.56 | (4a5,6S,8aS)-4a,5,9,10,11,12-Hexahydro-3-methoxy-6H-[1]benzofuro[3a,3,2-ef][2]benzazepin-6-ol |
| 14 | 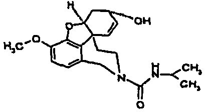 | (-) epi | > 100 | > 100 | (4aS,6S,8aS)-6-Hydroxy-3-methoxy-4a,5,9,10-tetrahydro-6H-[1]benzofuro[3a,3,2-ef][2]benzazepin-11(12H)-thiocarbonic acid methylamide |
| 15 | 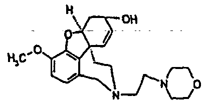 | (-)epi | 84 | > 100 | (4aS,6S,8aS)-4a,5,9,10,11,12-Hexahydro-11-(2-(morpholin-4-yl)ethyl)-3-methoxy-6H-[1]benzofuro[3a,3,2-ef][2]benzazepin-6-ol |
| 16 | 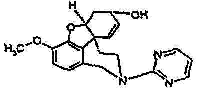 | (-)epi | > 100 | >100 | (4aS,6S,8aS)-4a,5,9,10,11,12-Hexahydro-3-methoxy-11-(2-pyrimidinyl)-6H-[1]benzofuro[3a,3,2-ef][2]benzazepin-6-ol |
| 17 | 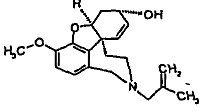 | (-) epi | >100 | >100 | (4aS,6S,8aS)-4a,5,9,10,11,12-Hexahydro-3-methoxy-11-(2-methyl-prop-2-enyl)-6H-[1]benzofuro[3a,3,2-ef][2]benzazepin-6-ol |
| 18 | 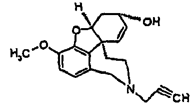 | (-) epi | >100 | >100 | (4aS,6S,8aS)-4a,5,9,10,11,12-Hexahydro-3-methoxy-11-propargyl-6H-[1]benzofuro[3a,3,2-ef][2]benzazepin-6-ol |
| 19 | 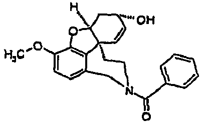 | (-) epi | >100 | >100 | (4aS,6S,8aS)-4a,5,9,10,11,12-Hexahydro-3-methoxy-11-benzoyl-6H-[1]benzofuro[3a,3,2-ef][2]benzazepin-6-ol |
| 20 | 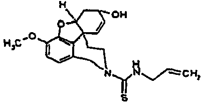 | (-) epi | >100 | >100 | (4aS,6S,8aS)-6-Hydroxy-3-methoxy-(4a,5,9,10-tetrahydro-6H-[1]benzofuro[3a,3,2-ef][2]benzazepin-11(12H)-thiocarbonic acid allylamide |
| 21 | 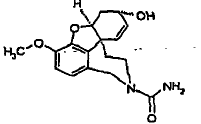 | (-) epi | >100 | > 100 | (4aS,6S,8aS)-4a,5,9,10-Tetrahydro-6-hydroxy-3-methoxy-6H- [1]benzofuro[3a,3,2-ef][2]benzazepin-11(12H)-carboxamid |
| 22 | 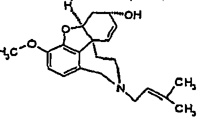 | (-) epi | 6 | 20 | (4aS,6S,8aS)-4a,5,9,10,11,12-Hexahydro-3-methoxy-11-(3-Methylbut-2-en-1-yl)-6H-[1]benzofuro[3a,3,2-ef][2]benzazepin-6-ol |
| 23 | 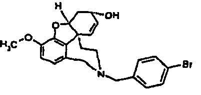 | (-) epi | 49 | 10 | (4aS,6S,8aS)-1-Bromo-11-(4-brombenzyl)-4a,5,9,10,11,12-Hexahydro-3-methoxy-6H-[1]benzefuro[3a,3,2-ef][2]benzazepin-6-ol |
| 24 | 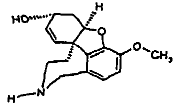 | (+) epi | >100 | >100 | (4aR,6R,8R)-4a,5,9,10,11,12-Hexahydro-3-methoxy-6H-[1]benzofuro[3a,3,2-ef][2]benzazepin-6-ol, |
| 25 | 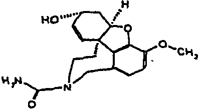 | (+) epi | >100 | >100 | (4aR,6R,8aR)-4a,5,9,10-Tetrahydro-6-hydroxy-3-methoxy-6H-[1]benzofuro[3a,3,2-ef][2]benzazepin-11(12H)-carboxamid |
| 26 | 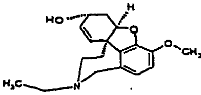 | (+) epi | >100 | > 100 | (4aR,6R,8aR)-4a,5,9,10,11,12-Hexahydro-3-methoxy-11-ethyl-6H-[1]benzofuro[3a,3,2-ef][2]benzazepin-6-ol |
| 27 | 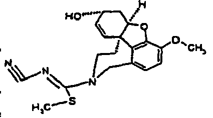 | (+) epi | >100 | >100 | Methyl (4aR,6R,6aR)-N11-cyano-6-hydroxy-3-methoxy-4a,5,9,10-tetrahydro-6H-[1]benzofuro[3a,3,2-ef][2]benzazepin-11 (12H)-carboximidothioal |
| 28 | 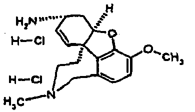 | (+) epi | >100 | >100 | (4aR,8R,8aR), 4a,5,9,10,11,12-Hexahydro-3. methoxy-11-methyl-6H-benzofuro[3a,3,2-ef][2]benzazepin-6-amin, Dihydrochloride |
| 29 | 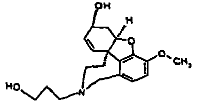 | (+) | > 100 | > 100 | (4aR,6S,8aR)-11-(3-hydroxypropyl)-3-methoxy-5,6,9,10,11,12-hexahydro-4aH-[1]benzofuro[3a,3,2-ef][2]benzazepin-6-ol |
| 30 | 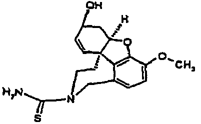 | (+) | > 100 | > 100 | (4aS,6R,8aS)-6-hydroxy-3-methoxy-5,6,9,10-tetrahydro-4aH-[1]benzofuro[3a,3,2-ef][2]benzazepine-11(12H)-carbothioamide |
| 31 | 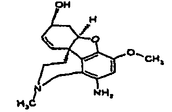 | (+) | >100 | >100 | (4aS,6S,8aS)-4a,5,9,10,11,12-Hexahydro-3-methoxy-11-benzoyl-6H-[1]benzofuro[3a,3,2-ef][2]benzazepin-6-ol |
| 32 | 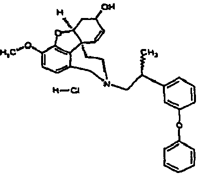 | (-) | 0,1 | 0,36 | 2-((4aS,6R,8aS)-6-Hydroxy-3-methoxy-5,6,9,10-tetrahydro-4aH-[1]benzofuro[3a,3,2-ef][2]benzazepin-11(12H)-yl)-1-methyl-1-(3-phenoxyphenyl)-ethane Hydrochloride |
| 33 | 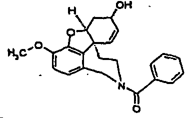 | (-) | 27 | >100 | (4aS,6R,8aS)-4a,5,9,10,11,12-Hexahydro-3-methoxy-11-benzoyl-6H-[1]benzofuro[3a,3,2-ef][2]benzazepin-6-ol |
| 34 | 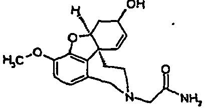 | (-) | 100 > 100 | 78 | 2-((4aS,6R,8aS)-6-Hydroxy-3-methoxy-5,6,9,10-tetrahydro-4aH-[1]benzofuro[3a,3,2-et][2]benzazepin-11(12H)-yl)acetamide |
| 35 | 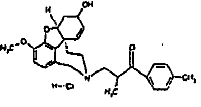 | (-) | 0,19 | 0,4 | 3-[(4aS,6R,8aS)-6-Hydroxy-3-methoxy-5,6,9,10-tetrahydro-4aH-[1]benzofuro[3a,3,2-ef][2]benzazepin-11(12H)-yl]-2-methyl-1(-4-methyl-phenyl)propan-1-on, Hydrochloride |
| 36 | 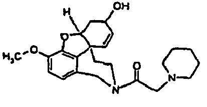 | (-) | 11 | > 100 | 1-[(4aS,6R,8aS)-6-hydroxy-3-methoxy-5,6,9,10-tetrahydro-4aH-[1]benzofuro[3a,3,2-ef][2]benzazepin-11(12H)-yl]-2-(1-piperidyl)ethane-1-on |
| 37 | 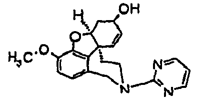 | (-) | >100 | >100 | (4aS,6R,8aS)-3-methoxy-11-(2-pyrimidinyl)-5,6,9,10,11,12-hexahydro-4aH-[1]benzofuro[3a,3,2-ef][2]benzazepin-6-ol |
| 38 | 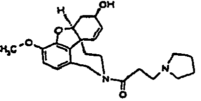 | (-) | 5,3 | 2,4 | 1-[(4aS,6R,8aS)-6-Hydroxy-3-methoxy-5,6,9,10-tetrahydro-4aH-[1]benzofuro[3a,3,2-ef][2]benzazepin-11(12H)-yl]-3-(1-pyrrolidyl)propan-1-on |
| 39 | 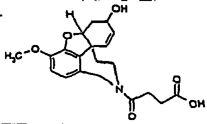 | (-) | >100 | >100 | ((4aS,8R,8aS)-4a,5,9,10,11,12-Hexahydro-Hexahydro-6-hydroxy-3-methoxy-6H-benzofuro[3a,3,2-ef][2]benzazepin-11-yl)gamma-oxo-butyric acid |
| 40 | 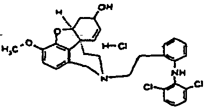 | (-) | 1,1 | 1,3 | 1-((4aS,6R,8aS)-6-Hydroxy-3-methoxy-5,6,9,10-tetrahydro-4aH-[1]benzofuro[3a,3,2, ef][2]benzazepin-11(12H)-yl)-2-[2-(2,6-dichloranilino)]-phenylethane |
| 41 | 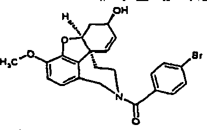 | (-) | 3,9 | 30 | (4aS,6R,8aS)-4a,5,9,10,11,12-Hexahydro-methoxy-11-(4-bromo-benzoyl)-6H-[1]benzofuro[3a,3,2-ef][2]benzezepin-6-ol |
| 42 | 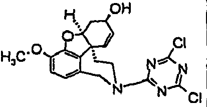 | (-) | > 100 | >100 | (4aS,6R,8aS)-11-(4,8-dichloro-1,3,5; triazin-2-yl)3-methoxy-5,6,9,10,11,12-hexahydro-4aH-[1]benzofuro[3a,3,2-ef][2]benzazepin-6-ol |
| 43 | 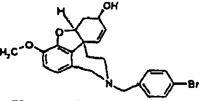 | (-) | 0,016 | 0,0006 | (4aS,6R,8aS)-11-(4-Brombenzyl)-4a,5,9,10,11,12-Hexahydro-3-methoxy-6H-[1]benzofuro[3a,3,2-ef][2]benzazepin-6-ol |
| 44 | 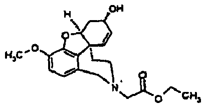 | (-) | 8,8 | 42 | Ethyl-2-((4aS,6R,8aS)-6-hydroxy-3-methoxy-5,6,9,10-tetrahydro-4aH-[1]benzofuro[3a,3,2-ef][2]benzazepin-11(12H)-yl)acetate |
| 45 | 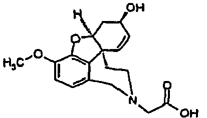 | (-) | | | 2-((4aS,6R,8aS)-6-Hydroxy-3-methoxy-5,6,9,10-tetrahydro-4aH-[1]benzefuro[3a,3,2-ef][2]benzazepin-11(12H)-yl)acetic acid |
| 46 | 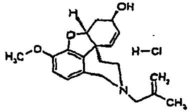 | (-) | 1,1 | 0,34 | (4aS,6R,8aS)-4a,5,9,10,11,12-Hexahydro-3-methoxy-11-(2-methy)-prop-2-enyl)-6H-[1]benzofuro[3a,3,2-ef][2]benzazepin-6-ol, Hydrochloride |
| 47 | 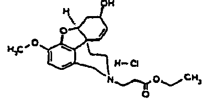 | (-) | 2,6 | 51 | Ethyl-3-((4a5,8R,8aS)-8-hydroxy-3-methoxy-5,6,9,10-tetrahydro-4aH-[1]benzofuro[3a,3,2-ef][2]benzazepin-11(12H)-yl)propanoate, Hydrochloride |
| 48 | 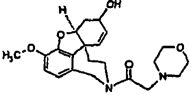 | (-) | 4,9 | 3,8 | 1-[(4aS-6R,8aS)-6-Hydroxy-3-methoxy-5,6,9,10-tetrahydro-4aH-[1]benzofuro[3a,3,2-ef][2]benzazepinn-11(12H)-yl]-2-(4-morpholinyl)ethan-1-on |
| 49 | 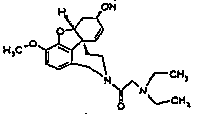 | (-) | 5,6 | 40 | 1-[(4aS,6R,8aS)-6-Hydroxy-3-methoxy-5,6,9,10-tetrahydro-4aH-[1]benzofuro[3a,3,2-ef][2]benzazepin-11(12H)yl]-2-(diethylamino)ethan-1-on |
| 50 | 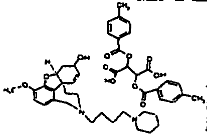 | (-) | 0,036 | 0,61 | (4aS,6R,8aS)-4a,5,9,10,11,12-Hexahydro-3-methoxy-11-[3-(1-pipendinyl)butyl]-6H-benzofuro[3a,3,2-ef][2]benzazepin-6-ol, (+) Di-O-p-toluoyl tartrate, |
| 51 | 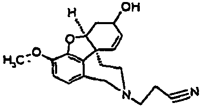 | (-) | 33 | 57 | 3-((4aS,6R,8aS)-1-bromo-hydroxy-3-methoxy-5,6,9,10-tetrahydro-4aH-[1]benzofuro[3a,3,2-ef][2]benzazepin-11(12H)-yl)propanenitrile |
| 52 | 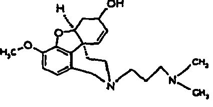 | (-) | 1,3 | 2,1 | (4aS,6R,8aS)-11-((3-dimethylamino)propyl)-3-methoxy-5,6,9,10,11,12-hexahydro-49H-[1]benzofuro[3a,3,2-ef][2]benzazepin-6-ol |
| 53 | 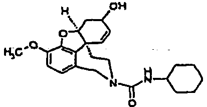 | (-) | 1,3 | >100 | (4aS,6R,8aS)-N11-cyclohexyl-6-hydroxy-3- methoxy-5,6,9,10-tetrahydro-4aH-[1]benzofuro[3a,3,2-ef][2]benzazepin-11(12H)-carbonoc acid isopropylamide |
| 54 | 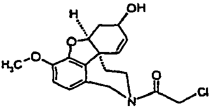 | (-) | 51 | > 100 | 1-[(4aS,6R,8aS)-6-hydroxy-3-methoxy-5,6,9,10-tetrahydro-4aH-[1]benzofuro[3a,3,2-ef][2]benzazepin-11(12H)-yl]-2-chlorethan-1-on |
| 55 | 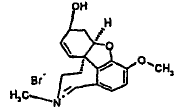 | (+) | >100 | >100 | (4aR, 6S, 8aR)-6-Hydroxy-3-methoxy-11-methyl-4a,5,9,10-tetrahydro-6H-benzofuro[3a,3, 2-ef][2]benzazepinium Bromide |
| 56 | 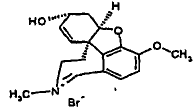 | (+) epi | >100 | >100 | (4aR, 6R, 8aR)-6-Hydroxy-3-methoxy-11-methyl-4a,5,9,10-tetrahydro-6H-benzofuro[3a,3, 2-ef][2]benzazepinium Bromide |
| 57 | 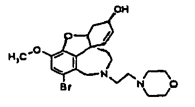 | (-) | >100 | 2.7 | (4aS,6R,8aS)-1-Bromo-4a,5,9,10,11,12-hexahydro-11-(2-(morpholin-4-yl)-ethyl)-3-methoxy-6H-[1]benzafuro[3a,3,2-ef][2]benzazepin-6-ol |
| 58 | 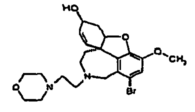 | (+) | >100 | 53 | (4aR,6R,8aRS)-1-Bromo-4a,5,9,10,11,12-hexahydro-11-(2-(morpholin-4-yl)-ethyl)-3-methoxy-6H-[1]benzofuro[3a,3,2-ef][2]benzazepin-6-ol |
| 59 | 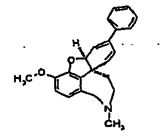 | (-) | 52 | 25 | (4aS,8aS)-D5,6-4a,5,9,10,11,12-Hexahydro-11-methyl-3-methoxy-6-phenyl-6H-[1]benzofuro[3a,3,2-et][2]benzazepine |
| 60 | 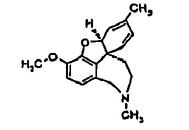 | (-) | 80 | 200 | (4aS,8aS)-D5,6-4a,5,9,10,11,12-Hexahydro-6,11-dimethyl-3-methoxy-6H-[1]benzofuro[3a,3,2-ef][2]benzazepine |
| 61 | 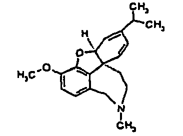 | (-) | >100 | 9 | (4aS,8aS)-D5,6-4a,5,9,10,11,12-Hexahydro-6-(isopropyl)-11-methyl-3-methoxy-6H-[1]benzofuro[3a,3,2-ef[2]benzazepine |

Die folgenden Beispiele zeigen mögliche erfindungsgemäße Synthesewege zur Bereitstellung der Verbindungen Ia, Ib und Ic:

### Beispiel 1

### (4aS,6S,8S)-1-Bromo-4a,5,9,10,11,12-hexahydro-3-methoxy-6H-[1]benzofuro[3a,3,2-ef][2]benzazepin-6-ol, (Ia Y₁=H, Y₂=OH, X=Br, Z₁=H)

20 g (-)-Brom-Norgalanthamin, welches gemäß der WO-A-97/40049 hergestellt wurde, werden in 800 ml 2 % HCl-Lösung unter Rückflusskühlung bei Siedetemperatur gerührt. Nach 3 Stunden wird die Reaktionsmischung abgekühlt, mit Ammoniak-Lösung basisch eingestellt und mit 3x300 ml Chloroform extrahiert. Die vereinigten org. Phasen werden über Natriumsulfat getrocknet. Das Trocknungsmittel wird abfiltriert und das Filtrat in Vakuum eingedampft.
Ausbeute: 14,9 g (75 % d. Th)
Smp: 198-203 °C
R_{f}: 0,25 (Chloroform:MeOH:Ammoniak-Lösung=90:9:1)
¹H-NMR (CDCl₃): δ 6.85 (s, 1H), 6.10 (d, 1H), 5.82 (d, 1H), 4.59 (m, 2H), 4.49 (d, 1H), 3.83 (d,1H), 3,80 (s, 3H), 3.30 (dd, 1H), 3.22 (dt, 1H), 2.72 (d, 1H), 1.88 (m, 2H), 1,71(t, 1H);
ATP-NMR (CDCl₃): δ146.8 (s) 143.9 (s) 139.2 (s), 132.4 4 (d), 131.5 (s), 126.3 (d), 115.4 (d), 112,5 (s), 88.6 (d), 62.7 (d), 56.2 (q), 52.4 (t), 49.2 (s), 46.9 (t) 40.6 (t), 32.1 (t);

### Beispiel 2

### (4aS,6S,8aS)-1-Bromo-6-hydroxy-3-methoxy-4a,5,9,10-tetrahydro-6H-[1]benzofuro[3a,3,2-ef][2]benzazepin-11(12H)-thiocarbonic acid allylamide (Ia Y₁=H, Y₂=OH, X=Br, Z₁=C₄H₅NS)

2,0 g (5,6 mmol) (-)-Epi-bromNorgalanthamin (Ia Y₁=H, Y₂=OH, X=Br, Z₁=H) werden in 60 ml Tetrahydrofuran gelöst, mit 0,6 ml Allylisothiocyanat versetzt und unter Rückflusskühlung bei 60 °C gerührt. Nach 40 Stunden wird das Lösungsmittel unter Vakuum abdestilliert und der Rückstand aus Chloroform n-Hexan kristallisiert.
Ausbeute: 2,28 g (76 % d. Th)
Smp: 199-207 °C
R_{f}: 0,75 (Chloroform:MeoH=9:1)
¹H-NMR (CDCl₃): δ 6.88 (s, 1H), 6.05 (d, 1H), 5.90 (m, 2H), 5.53 (d, 1H), 5.19 (d,1H), 5.10 (d, 1H), 4.64 (b, 2H), 4.50 (d, 1H), 4.31 (m, 1H), 4.19 (m,1H), 3,88 (s, 3H), 3.55 (t, 1H), 2.77 (m, 1H), 2.30 (t, 1H), 2.08 (b, 1H), 1.92 (d, 1H), 1,78(dt, 1H);
ATP-NMR (CDCl₃) : δ 180.8 (s), 148.2 (s) 145.7 (s) 134.3 (s), 133.9 (d), 133.5 (d), 225.7 (d), 225.3 (s), 117.9 (s), 115.4 (d), 112,3 (s), 89.0 (d), 63.3 (d), 56.7 (q), 52.0 (d), 49.5 (2t), 49.3 (s), 36.8 (t), 32.3 (t);

### Beispiel 3

### (4aR,6R,8aR)-1-Bromo-4a,5,9,10,11,12-hexahydro-3-methoxy-6H-[1]benzofuro[3a,3,2-ef] [2]benzazepin-6-ol (Ib Y₁=H, Y₂=OH, X=Br, Z₁=H)

20 g (+)-Brom-Norgalanthamin, welches gemäß der WO-A-97/40049 hergestellt wurde, werden in 800 ml 2 % HCl-Lösung unter Rückflusskühlung bei der Siedetemperatur gerührt. Nach 3 Stunden wird die Reaktionsmischung abgekühlt, mit cc Ammoniak-Lösung basisch eingestellt und mit 3x300 ml Chloroform extrahiert. Die vereinigten organischen Phasen werden über Natriumsulfat getrocknet. Das Trocknungsmittel wird abfiltriert und das Filtrat in Vakuum eingedampft.
Ausbeute: 15,5 g (78 % d. Th)
Smp: 103-205 °C
R_{f}: 0,25 (Chloroform:MeOH:Ammoniak-Lösung=90:9:1)
¹H-NMR (CDCl₃): δ 6.88 (s, 1H), 6.07 (d, 1H), 5.82 (d, 1H), 4.59 (m, 2H), 4.51 (d, 1H), 3.83 (d,1H), 3,80 (s, 3H), 3.28 (d, 1H), 3.22 (t, 1H), 2.78 (d, 1H), 1. 91 (m, 2H), 1,73 (t, 1H) ;
ATP-NMR (CDCl₃) : δ 146.8 (s) 143.9 (s) 134.2 (s), 132.4 4 (d), 131.5 (s), 126.3 (d), 115.4 (d), 112,5 (s), 88.6 (d), 62.7 (d), 56.2 (q), 52.4 (t), 49.2 (s), 46.9 (t) 40.6 (t), 32.1 (t);

### Beispiel 4

### (4aR,6R,8aR)-1-Bromo-4a,5,9,10-tetrahydro-6-hydroxy-3-methoxy-6H-[1]benzofuro-[3a,3,2-ef][2]benzazepin-11(12H)-thiocarbonic acid methylamide (Ib Y₁=H, Y₂=OH, X=Br, Z₁=C₂H₄NS)

1,96 g (+)-Epi-bromNorgalanthamin (Ib Y₁=H, Y₂=OH, X=Br, Z₁=H) und 0,7 g Methylisothiocyanat werden in 50 ml Toluol bei Rückflusstemperatur gerührt. Nach 16 Stunden wird die Reaktionsmischung auf Raumtemperatur abgekühlt, das Lösungsmittel unter Vakuum abdestilliert und der Rückstand mit 200 ml 2N HCl und mit 50 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=95:5) gereinigt.
Ausbeute: 1,4 g (54 % d. Th)
Smp: 80-88°C
R_{f}: 0,45 (Chloroform:MeOH:Ammoniak-Lösung=90:9:1)
¹H-NMR (DMSO): δ 8.35 (b, 1H, N-H), 6.99 (s, 1H), 6.01 (b, 1H), 5.72 (d, 1H), 4.98 (d, 1H), 4.61 (b, 1H), 4.29(m, 1H), 3.79 (d,1H), 3,74 (s, 3H), 2.90 (d, 3H), 2.51 (d, 1H), 2.48 (t, 1H), 1 . 98 (t, 1H), 1.81 (m, 2H), 1,65 (t, 1H) ;
ATP-NMR (DMSO): δ 182.0 (s), 147.7 (s) 144.6 (s) 134.4 (s), 133.8 (d), 128.6 (s), 126.8 (d), 116.4 (d), 113,5 (s), 88.5 (d), 62.0 (d), 56.8 (q), 49.2 (t), 40.0 (s), 36.5 (t) 33.7 (q), 32.4 (t);

### Beispiel 5

### 1-[(4aR,6S,8aR)-6-Hydroxy-3-methoxy-5,6,9,10-tetrahydro-4aH-[1]benzofuro[3a,3,2-ef][2]benzazepin-11(12H)-yl]-3-(1-pyrrolidyl)propan-1-on (Ib Y₁=OH, Y₂=H, X=Br, Z₁=C₇H₁₂NO)

2,0 g (+)-BromNorgalanthamin , welches gemäß der WO-A-97/40049 hergestellt wurde, sowie 1,0 ml Triethylamin und 0,6 ml 3-Brompropionsäurechlorid werden in 100 ml Tetrahydrofuran bei 0°C gerührt. Nach 10 min wird die Reaktionsmischung mit 1,6 g Kaliumcarbonat und 0,6 ml Pyrrolidin versetzt und bei 90 °C weitergerührt. Nach 17 Stunden wird das Lösungsmittel abdestilliert, der Rückstand mit 50 ml Wasser und 50 ml Chloroform versetzt. Nach dem Abtrennen der organischen Phase wird die wässrige Phase mit 2x50 ml Chloroform extrahiert. Die vereinigten organischen Phasen werden über Natriumsulfat getrocknet, filtriert und unter Vakuum eingeengt. Das Produkt wird säulenchromatographisch (Chloroform:MeOH:Ammoniak-Lösung=90:9:1) gereinigt.
Ausbeute: 1,88 g (69,3 % d. Th)
Smp: 80-85 °C
R_{f}: 0,4 (Chloroform:MeOH:Ammoniak-Lösung=90:9:1)
¹H-NMR (CDCl₃): δ 6.90 (s, 1H), 6.07 (dd, 1H), 5.18 (d, 1H), 4.59 (m, .2H), 4.31 (d, 1H), 4.18 (m,1H), 3,80 (s, 3H), 3.78 (d, 1H), 3.22 (t, 1H), 2.90 (m, 3H), 2.68 (m, 3H), 2.6-2.35 (m, 8H), 2.01 (dd, 1H), 1,89 (dt, 1H) ;
ATP-NMR (CDCl₃): δ 171.4 (s), 146.5 (s), 144.9 (s), 133.6 (s), 128.8 (d), 127.3 (s), 126.0 (d), 115.7 (d), 112,8 (s), 88.3 (d), 61.6 (d), 56.2 (q), 54.2 (2t), 51.8 (t), 51.4 (t) 49.0 (s), 44.5 (t), 35.6 (t), 33.0 (t), 29.6 (t)23.4 (2t);

### Beispiel 6

### 4aR,6S,8aR)-11-Benzyl-1-bromo-4a,5,9,10,11,12-Hexahydro-3-methoxy-6H-[1]benzofuro[3a,3,2-ef][2]benzazepin-6-ol (Ib Y₁=OH, Y₂=H, X=Br, Z₁=C₇H₇)

2,0 g (+)-BromNorgalanthamin, welches gemäß der WO-A-97/40049 hergestellt wurde, sowie 4,0 g Kaliumcarbonat und 0,71 ml Benzylbromid werden in 40 ml Acetonitril bei Rückflusstemperatur gerührt. Nach 3 Stunden wird die Reaktionsmischung auf Raumtemperatur abgekühlt, das Lösungsmittel unter Vakuum abdestilliert und der Rückstand mit 60 ml Wasser und 50 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=99:1) gereinigt.
Ausbeute: 1,76 g (69,8 % d. Th) gelbes Öl
R_{f}: = 0,75 (Chloroform:MeOH=:99:1)
¹H-NMR (DMSO) : δ 7.28 (m, 5H), 6.92 (s, 1H), 6.18 (d, 1H), 5. 85 (dd, 1H), 4.59 (b, 1H), 4.35 (d, 1H), 4.12 (m, 2H), 3,78 (s, 3H), 3.64 (d, 1H), 3.55 (d, 1H), 2.98 (d, 1H), 2.52 (s, 2H), 2.27 (d, 1H), 2,09 (m, 2H);
ATP-NMR (DMSO) : δ 146.9 (s), 144.6 (s), 139.7 (s) 135.0 (s), 129.5 (d), 129.5 (2d), 129.0 (2d), 128.7 (s), 127.7 (d), 127.4 /d), 116.3 (d), 113,3 (s), 87.7 (d), 60.5 (d), 56.7 (q), 56.4(t) 51.2 (t), 49.3 (s), 39.9 9 (t) 34.2 (t), 31.6 (t);

### Beispiel 7

### 1-[(4aR,6S,8aR)-1-Bromo-6-hydroxy-3-methoxy-5,6,9,10-tetrahydro-4aH-[1]benzofuro[3a,3,2-ef][2]benzazepin-11(12H)-yl]-2-(4-methylpiperazinyl) ethan-1-on (Ib Y₁=OH, Y₂=H, X=Br, Z₁=C₇H₁₃N₂O)

2,0 g (+)-BromNorgalanthamin, welches gemäß der WO-A-97/40049 hergestellt wurde, sowie 3,92 g Kaliumcarbonat werden in 50 ml Tetrahydrofuran gerührt und die Suspension mittels Eisbad auf 0°C gekühlt. Nach dem Zutropfen von 0,48 ml Chloracetylchlorid wird noch 30 min bei 0°C gerührt und danach 1,4 ml N-Methylpiperazin zugegeben. Nach 48 Stunden Rückfluss wird abkühlen gelassen, 150 ml Wasser zugegeben und mit 3x40 ml Ethylacetat ausgeschüttelt. Die vereinigten organischen Phasen werden mit Natriumsulfat getrocknet und eingedampft. Der Rückstand wird säulenchromatographisch (Chloroform:MeOH:Ammoniak-Lösung=95:4,5:0,5) gereinigt.
Ausbeute: 0,7 g (17,9 % d. Th.) weißer Schaum
R_{f}: 0,42 (Chloroform:MeOH:Ammoniak-Lösung =90:9:1)
¹H-NMR (CDCl₃): δ 6.88 (s, 1H), 6.05 (dd, 1H), 5.94 (d, 1H), 5.59 (d, 1H), 4.58 (b, 1H), 4.31 (d, 1H), 4.12 (t, 1H), 3.85 (s, 3H), 3,83 (d, 1H), 3.30 (d, 1H), 3.21 (m, 1H), 3.03 (d, 1H), 2.71 (d, 1H), 2.42 (m, 8H), 2.29 (s, 3H), 2.04 (dd, 1H), 1,95 (dd, 1H), 1,78 (d, 1H) ;
ATP-NMR (CDCl₃): δ 169.9 (s), 146.9 (s) 144.9 (s) 133.4 (s), 129.1 (d), 128.3 (s), 126.6 (d), 116.3 (d), 113.4 (s), 88.8 (d), 62.0 (d), 61.4 (t) 56.6 (q), 55.4 (2t), 53.7 (2t), 52.0 (s), 49.5 (t), 46.6 (q), 45.2 (t), 35.9 (t), 30.1 (t);

### Beispiel 8

### (4aR,6R,8aR)-11-(3-(4-methylpiperazine)-1-yl-propyl)-3-methoxy-5,6,9,10,11,12-hexahydro-4aH-[1]benzofuro[3a,3,2-ef][2]benzazepin-6-ol, Trihydrochloride (Ib Y₁=OH, Y₂=H, X=Br, Z₁=C₇H₁₇N₂)

### Stufe 1

2g (+)-BromNorgalanthamin, welches gemäß der WO-A-97/40049 hergestellt wurde, sowie 5,6 ml 1-Brom-3-Chlorpropan und 3,92g Kaliumcarbonat werden in 10 ml Acetonitril bei 80 °C 4,5 Stunden lang gerührt. Nach der Filtration des Kaliumcarbonats werden 70 ml Wasser zugesetzt, mit 2N HCl angesäuert und 2mal mit je 30 ml Ethylacetat extrahiert. Die wässrige Phase wird mit 2N Natriumhydroxid-Lösung basisch eingestellt und 2mal mit je 50 ml Dichlormethan ausgeschüttelt. Nach dem Abtrennen des Lösungsmittels verblieben 1,12g (46 % d. Th) gelbliches Öl. Das Produkt wurde sofort weiterverarbeitet.

### Stufe 2

1,1g N-(3-Chlorpropyl)-(+)-Bromnorgalanthamin (Stufe 1), 2,85 ml N-Methylpiperazin und 2,1 g Kaliumcarbonat werden in 8 ml Acetonitril bei 90 °C 3 Stunden lang gerührt. Das Kaliumcarbonat wird abfiltriert, und die Lösung eingedampft. Die erhaltenen 2,27 g werden an 170 g Kieselgel mit dem Laufmittel Chloroform:Methanol:Ammoniak-Lösung=90:9:1 gesäult. Die produkthaltigen Fraktionen werden eingedampft, der Rückstand in 15 ml Ether gelöst und bei 0 °C mit etherischer HCl angesäuert. Nach dem Abfiltrieren und zweimaligem Waschen mit je 5 ml Ether wird das Produkt bei 30 °C im Vakuumtrockenschrank bei 50 mbar 16 Stunden getrocknet.
Ausbeute: 440 mg (32 % d. Th.) weiße Kristalle
Smp: 220-238 °C
R_{f}: 0,37 (Chloroform:Methanol:Ammoniak-Lösung=90:9:1)
¹H-NMR (DMSO): δ 7.19 (s, 1H), 6.21 (d, 1H), 5.89 (d, 1H), 4.89 (d, 1H), 4.70 (b, 1H), 4.55 (d, 1H), 4.09 (b, 1H), 3.81 (s, 3H), 3,01-3.80 (m, 12H), 2.80 (m, 3H), 2.02 (s, 3H), 2.31 (m, 2H), 2.08 (m, 2H) 1, 85 (b, 1H) ;
ATP-NMR (DMSO): δ 172.7 (s), 147.3 (s) 146.3 (s) 134.8 (s), 132.1 (d), 131.9 (d), 125.7 (d), 117.1 (d), 115.0 (s), 87.4 (d), 65.7 (2t), 65.0 (2t), 60.1 (d), 53.2 (q), 49.9 (d), 48.2 (d), 43.7 (s), 42.8 (q), 41.1 (d), 41,0 (d), 40,9 (d), 31.4 (t);

### Beispiel 9

### Methyl-4-((4aR,6S,8aR)-1-Bromo-4a,5,9,10,11,12-hexahydro-6-hydroxy-3-methoxy-6H-benzofuro[3a,3,2-ef] [2] benzazepin-11-yl)gamma-oxo-butyrate (Ib Y₁=OH, Y₂=H, X=Br, Z₁=C₅H₇O₃)

### Stufe 1:

2,0 g (+)-BromNorgalanthamin, welches gemäß der WO-A-97/40049 hergestellt wurde, sowie 1,18 ml Triethylamin und 0,6 g Bernsteinsäureanhydrid werden in 70 ml Tetrahydrofuran bei 75°C intensiv gerührt. Nach 30 min wird die Reaktionsmischung abgekühlt, das Lösungsmitten im Vakuum abdestilliert, und der Rückstand mit 100 ml 2N HCl und mit 50 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und im Vakuum eingeengt.
Ausbeute: 1,91 g gelblicher Schaum

### Stufe 2:

1,91 g Schaum, wie gemäß Stufe 1 hergestellt, werden in 20 ml Methanol aufgelöst, mit 0,6 ml Dimethylsulfat versetzt und bei Raumtemperatur (RT) gerührt. Nach 24 Stunden wird das Reaktionsgemisch mit 50 ml Wasser und mit 40 ml Ethylacetat versetzt. Nach dem Abtrennen der organischen Phase wird die wässrige Phase mit 2x30 ml Ethylacetat extrahiert. Die vereinigten organischen Phasen werden über Natriumsulfat getrocknet, filtriert und im Vakuum eingeengt. Das Produkt wird säulenchromatographisch (Ethylacetat) gereinigt.
Ausbeute: 0,54 g (24,3 % d. Th) farbloses Öl
R_{f}: 0,35 (Ethylacetat)
¹H-NMR (DMSO): δ 7.25 (s, 1H), 6.12 (d, 1H), 5.81 (m, 1H), 5.01 (d, 1H), 4.69 (d, 1H), 4.51 (d, 1H), 4.42 (m, 1H), 4.09 M, 1H), 3.78 (s, 3H), 3.53 (s, 3H), 3.29 (m, 1H), 2.77 (m, 1H), 2.52 (m, 3H), 2.28 (d, 1H), 2.04 (m, 1H), 1, 85 (m, 1H), 1.69 (m, 1H);
ATP-NMR (DMSO): δ 173.6 (s), 170.7 (s), 147.4 (s), 144.7 (s), 134.3 (s), 129.6 (d), 128.3 (s), 127.2 (d), 116.2 (d), 111.9 (s), '87.4 (d), 60.2 (d), 56.8 (q), 52.0 (q), 51.2 (t), 49.3 (s), 46.2 (t) 39.9 (t),37.0 (t), 31.2 (t), 28.7 (t);

### Beispiel 10

### (4aR,6S,8aR)-11-(4-aminopropyl)-3-methoxy-5,6,9,10,11,12-hexahydro-4aH-[1]benzofuro[3a,3,2-ef] [2]benzazepin-6-ol, Methansulfonate (Ib Y₁=OH, Y₂=H, X=Br, Z₁=C₃H₈N)

### Stufe 1:

2,0 g (+)-BromNorgalanthamin, welches gemäß der WO-A-97/40049 hergestellt wurde, sowie 7,2 ml 1-Brom-3-chlorpropan und 5,0 g Kaliumcarbonat werden in 10 ml Acetonitril bei Raumtemperatur gerührt. Nach 19 Stunden wird der Niederschlag abfiltriert, und das Filtrat mit 80 ml Wasser, 25 ml 2N HCl und 50 ml Ethylacetat versetzt. Nach dem Abtrennen der organischen Phase wird die wässrige Phase mit 2x40 ml Ethylacetat extrahiert. Die vereinigten organischen Phasen werden über Natriumsulfat getrocknet, filtriert und im Vakuum eingeengt.
Ausbeute: 1,5 g farbloser Schaum.

### Stufe 2:

1,5 g Schaum, wie in Stufe 1 hergestellt, werden in 15 ml Methanol aufgelöst, mit 15 g NH₄Cl, mit 150 ml 25%er Ammoniak-Lösung versetzt und bei Raumtemperatur gerührt. Nach 18 Stunden wird die Reaktionsmischung mit 400 ml Wasser und 75 ml Chloroform versetzt. Nach dem Abtrennen der organischen Phase wird die wässrige Phase mit 2x75 ml Chloroform extrahiert. Die vereinigten organischen Phasen werden über Natriumsulfat getrocknet, filtriert und unter Vakuum eingeengt. Der Rückstand wird in 5 ml Tetrahydrofuran aufgelöst und mit Methansulfonsäure bis pH 1 angesäuert. Der entstandene Niederschlag wird abgetrennt, mit Tetrahydrofuran nachgewaschen und im Vakuumtrockenschrank getrocknet.
Ausbeute: 1,3 g (71 % d. Th)
Smp: 63-67 °C
R_{f}: 0,15 (Chloroform:MeOH:Ammoniak-Lösung = 0:18:2)
¹H-NMR (DMSO): δ 7.88 (b, 2H, NH₂), 6.85 (s, 1H), 6.19 (d, 1H), 5.89 (d, 1H), 4.70 (d, 1H), 4.60 (b, 1H), 4.48 (b, 1H), 4.18 (m, 1H), 3.80 (s, 3H), 3.70 (m, 1H), 3.59 (b, 2H), 3.42 (m, 2H), 2.88 (b, 2H), 2.52 (m, 1H), 1.91- 2.34 (m, 4H);
ATP-NMR (DMSO): δ 147.3 (s) 145.6 (s) 133.6 (s), 130.4 (d), 126.5 (s), 123.6 (d), 112.9 (d), 112,1 (s), 87.3 (d), 67.9 (2t), 60.4 (d), 56.5 (q), 49.8 (t), 40.9 (t), 40.8 (s), 37.3 (t) 31.7 (t), 25.9 (t);

### Beispiel 11

### (4aR,6S,8aR)-1-Bromo-4a,5,9,10-tetrahydro-6-hydroxy-3-methoxy-6H-[1] benzofuro-[3a,3,2-ef] [2] benzazepine-11(12H)-thiocarbonic acid methylamide (Ib Y₁=OH, Y₂=H, X=Br, Z₁=C₂H₄NS)

2,0 g (+)-BromNorgalanthamin, welches gemäß der WO-A-97/40049 hergestellt wurde, sowie 0,7 g Methylisothiocyanat werden in 50 ml Toluol bei Rückflusstemperatur gerührt. Nach 16 Stunden wird die Reaktionsmischung auf Raumtemperatur abgekühlt, das Lösungsmittel unter Vakuum abdestilliert, und der Rückstand mit 200 ml 2N HCl und 50 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=95:5) gereinigt.
Ausbeute: 2,2 g (93 % d. Th)
Smp: 98-102°C
R_{f}: 0,7 (Chloroform:MeOH:Ammoniak-Lösung = 90:9:1)
¹H-NMR (DMSO): δ 7.38 (b, 1H, NH), 6.97 (s, 1H), 6.07 (d, 1H), 5.80 (dd, 1H), 4.51 (b, 1H), 4.37 (m, 2H), 4.09 (d, 1H), 3.80 (m, 1H), 3,72 (s, 3H), 3.31 (b, 1H), 2.81 (s, 3H), 2.28 (d, 1H), 2.02 (d, 1H), 1.85 (t, 1H), 1, 61 (d, 1H);
ATP-NMR (DMSO) : δ 182.0 (s), 147.5 (s) 144.7 (s) 134.0 (s), 129.4 (d), 128.6 (s), 127.6 (d), 116.4 (d), 113.2 (s), 87.3 (d), 60.2 (d), 56.8 (q), 57.1 (t), 49.1 (s), 48.4 (t) 36.7 (t), 33.7 (q), 31.2 (t);

### Beispiel 12

### (4aS, 6R, 8aS)-1-Bromo-6-hydroxy-3-methoxy-4a,5,9,10-tetrahydro-6H-[1]benzofuro[3a,3,2-ef][2]benzazepin-11(12H)-thiocarbonic acid allylamide (Ia Y₁=OH, Y₂=H, X=Br, Z₁=C₄H₆NS)

2,0 g (-)-BromNorgalanthamin, welches gemäß der WO-A-97/40049 hergestellt würde, sowie 0,6 ml Allylisothiocyanat werden in 60 ml Tetrahydrofuran bei Rückflusstemperatur gerührt. Nach 9 Stunden wird die Reaktionsmischung auf Raumtemperatur abgekühlt, das Lösungsmittel unter Vakuum abdestilliert, und der Rückstand säulenchromatographisch (Chloroform:MeOH=98:2) gereinigt.
Ausbeute: 2,14 g (83,5 % d. Th)
Smp: 175-179 °C
R_{f}: 0,45 (Chloroform:MeOH=:9:1)
¹H-NMR (DMSO): δ 7.41 (b, 1H, NH), 6.95 (s, 1H), 6.06 (d, 1H), 5.76 (m, 2H), 5.08 (t, 2H), 4.42 (m, 3H), 4.09 (m, 2H), 3.83 (m,1H) 3,72 (s, 3H), 2,51 (d, 2H), 2.28 (d, 1H), 2.05 (d, 1H), 1.88 (t, 2H), 1,76(t, 1H);
ATP-NMR (DMSO): δ181.3 (s), 147.5 (s) 144.8 (s), 136.1 (d), 134.0 (s), 129.4 (d), 128.2 (s), 127.5 (d), 116.3 (d), 116.3 (s), 113,1 (d), 87.3 (d), 60.2 (d), 56.8 (q), 49.0 (t), 48.8 (s), 41.0 (t) 40.9 (t), 36.7 (t), 31.1 (t);

### Beispiel 13

### (4aS,6S,8aS)-4a,5,9,10,11,12-Hexahydro-3-methoxy-6H-[1]benzofuro[3a,3,2-ef][2]benzazepin-6-ol (Ia Y₁=H, Y₂=OH, X=H, Z₁=H)

1g (-)-Norgalanthamin , welches gemäß der W-A-00/174820 hergestellt wurde, wird in 80 ml 2 %-iger HCI gelöst und 3 Stunden bei Rückfluss gerührt. Das Reaktionsgemisch auf Raumtemperatur abkühlen gelassen, mit konzentrierter, wässriger Ammoniak-Lösung basisch eingestellt und 3x mit je 30 ml Chloroform extrahiert. Die vereinigten organischen Phasen werden über Natriumsulfat getrocknet, filtriert und eingedampft. Die erhaltenen 1,06 g werden an 80 g Kieselgel mit dem Laufmittel Chloroform:Methanol:-Ammoniak-Lösung=90:9:1 säulenchromato-graphisch gereinigt, und die erhaltenen, produkthältigen Fraktionen eingedampft.
Ausbeute: 690 mg (69 % d. Th.) weißes Pulver
Smp: 151-155
R_{f}: 0,29 (Chloroform:Methanol:Ammoniak-Lösung=90:9:1)
¹H-NMR (CDCl₃): δ 6.65 (d, 1H), 6.49 (d, 1H), 6.02 (d, 1H), 5.65 (d, 1H), 4.48 (b, 1H), 4.25 (m, 1H), 3.85 (d, 1H), 3.74 (d, 1H), 3,65 (s, 3H), 3.15 (dd, 1H), 3.03 (m, 1H), 2.43 (m, 1H), 1.79 (m, 2H), 1,63 (t, 1H);
ATP-NMR (CDCl₃) δ 147.6 (s),143.8 (s), 135.1 (s), 134.2 (s), 133.3 (d), 127.3 (d), 120.3 (d), 112.0 (d), 88.7 (d), 62.2 (d), 56.4 (q), 53.8 (t), 49.0 (s), 47.5 (t) 39.7 (t), 32.9 (t);

### Beispiel 14

### (4aS,6S,8aS)-6-Hydroxy-3-methoxy-4a,5,9,10-tetrahydro-6H-[1]benzofuro [3a,3,2-ef][2]benzazepin-11(12H)-thiocarbonic acid methylamide (Ia Y₁=H, Y₂=OH, X=H, Z₁=C₄H₈NO)

2,32 g (-)-Epi-Norgalanthamin (Ia Y₁=H, Y₂=OH, X=H, Z₁=H) und 0,9 ml Isopropylisocyanat werden in 150 ml Toluol bei Rückflusstemperatur gerührt. Nach 16 Stunden wird die Reaktionsmischung auf Raumtemperatur abgekühlt, das Lösungsmittel unter Vakuum abdestilliert, und der Rückstand mit 200 ml 2N HCl und 50 ml Ethylacetat versetzt. Nach dem Abtrennen der organischenPhase wird die wässrige Phase mit 2x50 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=98:2) gereinigt.
Ausbeute: 1,49 g (49,1 % d. Th)
Smp:182-186 °C
R_{f}: 0,5 (Chloroform:MeOH:Ammoniak-Lösung=90:9:1)
¹H-NMR (CDCl₃): δ 6.69 (b, 2H), 5.98 (d, 1H), 5.81 (d, 1H), 4.61 (m, 2H), 4.48 (d, 1H), 4.32 (d, (1H), 4.21 (d, 1H), 3,89 (m, 1H), 3.87 (s, 3H), 3.42 (t, 1H), 2.79 (d, 1H), 2.01 (dt, 1H), 1.79 (dd, 1H), 1.61(d, 1H), 1.11 (d, 3H), 0,98 (d, 3H);
¹³C-NMR (CDCl₃): δ 157.0 (s), 148.3 (s) 144.8 (s) 132.9 (d), 132.8 (s), 129.4 (d), 126.4 (d), 120.0 (d), 111.3 (d), 88.8 (d), 63.3 (d), 56.3 (q), 51.8 (t), 48.8 (s), 46.0 (t), 42.9 (d), 37.7 (t), 32.6 (t), 23.9 (q), 23.6 (q);

### Beispiel 15

### (4aS,6S,8aS)-4a,5,9,10,11,12-Hexahydro-11-(2-(morpholin-4-yl)-ethyl)-3-methoxy-6H-[1]benzofuro[3a,3,2-ef][2]benzazepin-6-ol (Ia Y₁=H, Y₂₌OH, X=H, Z₁=C₆H₁₂NO)

1,55 g (-)-Epi-Norgalanthamin (Ia Y₁=H, Y₂=OH, X=H, Z₁=H), 2,35 g Kaliumcarbonat und 1,11 g N-(2-Chlorethyl)-morpholin Hydrochlorid werden in 30 ml Acetonitril bei Rückflusstemperatur gerührt. Nach 48 Stunden wird die Reaktionsmischung auf Raumtemperatur abgekühlt, das Lösungsmittel im Vakuum abdestilliert, und der Rückstand mit 200 ml 2N HCl und 40 ml Ethylacetat versetzt. Nach dem Abtrennen wird die organische Phase entsorgt. Die wässrige Phase wird mit Ammoniak-Lösung basisch eingestellt und mit 3x40 ml Ethylacetat extrahiert. Die vereinigten organischen Phasen werden über Natriumsulfat getrocknet, filtriert und unter Vakuum eingeengt. Das Produkt wird säulenchromatographisch (Chloroform:-MeOH=9:1) gereinigt.
Ausbeute: 0,51 g (23,3 % d. Th) weißer Schaum
R_{f}: 0,5 (Chloroform:MeOH=:9:1)
¹H-NMR (CDCl₃): δ 6.69 (d, 1H), 6.57 (d, 1H), 6.07 (d, 1H), 5.78 (d, 1H), 4.61 (m, 2H), 4.18 (d, 1H), 4.35 (s, 3H), 3.80 (d, 1H), 3,65 (m, 4H), 3.31 (t, 1H), 3.09 (d, 1H), 2.69 (d, 1H), 2.57 (m, 2H), 2.50 (m, 5H), 2.28 (b, 1H), 2.19 (t, 1H), 1.72 (t, 1H), 1.59 (d, 1H) ;
¹³C-NMR (CDCl₃): δ 146.7 (s) 143.9 (s) 133.0 (s), 131.8 (d), 129.4 (d), 126.4 (d), 121.5 (d), 110.9 (d), 88.4 (d), 66.8 (2t), 63.0 (d), 57.7 (d), 57.1 (q), 55.8 (t), 54.1 (2t), 52.1 (s), 48.3 (t), 47.9 (t), 33.5 (t), 32.4 (t);

### Beispiel 16

### (4aS,6S,8aS)-4a,5,9,10,11,12-Hexahydro-3-methoxy-11-(2-pyrimidinyl)-6H-[1]benzofuro[3a,3,2-ef][2]benzazepin-6-ol (Ia Y₁=H, Y₂=OH, X=H, Z₁=C₃H₃N₂)

2,0 g (-)-Epi-Norgalanthamin (Ia Y₁=H, Y₂=OH, X=H, Z₁=H), 2,45 g NaHCO₃ und 0,88 g 2-Chlorpyrimidin werden in 120 ml Ethanol bei Rückflusstemperatur gerührt. Nach 44 Stunden wird die Reaktionsmischung auf Raumtemperatur abgekühlt, das Lösungsmittel im Vakuum abdestilliert und der Rückstand mit 120 ml Wasser und 200 ml Ethylacetat versetzt. Nach dem Abtrennen der organischen Phase wird die wässrige Phase mit 2x100 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=98:2) gereinigt.
Ausbeute: 1,24 g (48,2 % d. Th)
Smp: 223-226 °C
R_{f}: 0,65 (Chloroform:MeOH=9:1)
¹H-NMR (DMSO): δ 8.30 (d, 2H), 6.72 (d, 1H), 6.65 (d, 1H), 6.54 (t, 1H), 6.20 (d, 1H), 5.72 (d, 1H), 5.29 (d, 1H), 5.08 (d, 1H), 4.79 (d, 1H), 4.48 (m, 2H), 4.25 (m, 1H), 3.68 (s, 3H), 2.45 (m, 1H), 1.95 (t, 1H), 1.78 (d, 1H), 1.65 (t, 1H);
¹³C-NMR (DMSO) : δ 161.1 (s), 158.8 (s), 147.9 (s), 144.1 (s) 133.6 (s), 133.5 (2d), 130.6 (d), 126.9 (d), 122.1 (d), 111.9 (d), 110.7 (d), 88.4 (d), 62.2 (d), 56.4 (q), 48.8 (t), 45.5 (s), 41.0 (t), 36.5 (t), 32.8 (t);

### Beispiel 17

### (4aS,6S,8aS)-4a,5,9,10,11,12-Hexahydro-3-methoxy-11-(2-methyl-prop-2-enyl)-6H-[1]benzofuro[3a,3,2-ef][2]benzazepin-6-ol (I Y₁=H, Y₂=OH, X=H, Z₁=C₄H₇)

2,0 g (-)-Epi-Norgalanthamin (Ia Y₁=H, Y₂=OH, X=H, Z₁=H), 2,02 g Kaliumcarbonat, 1,27 g Kaliumjodid und 0,85 ml 3-Chlor-2-methyl-1-propen werden in 80 ml Aceton bei Rückflusstemperatur gerührt. Nach 48 Stunden wird die Reaktionsmischung auf Raumtemperatur abgekühlt, das Lösungsmittel unter Vakuum abdestilliert und der Rückstand mit 200 ml 2N HCl und 50 ml Ethylacetat versetzt. Nach dem Abtrennen wird die organische Phase entsorgt. Die wässrige Phase wird mit 30%-iger Natriumhydroxid-Lösung basisch eingestellt und mit 3x100 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=95:5) gereinigt.
Ausbeute: 1,8 g (75,1 % d. Th) harziges Öl
R_{f}: 0,65 (Chloroform:MeOH=95:5)
¹H-NMR (CDCl₃) : δ 6.72 (d, 1H), 6.53 (d, 1H), 6.11 (d, 1H), 5.82 (d, 1H), 4.85 (d, 2H), 4.60 (m, 1H), 4.54 (b, 1H), 4.09 (d, 1H), 3.87 (s, 3H), 3.67 (d, 1H), 3,32 (t, 1H), 3.05 (m, 2H), 2.83 (d, 1H), 2.18 (dt, 1H), 1.93 (b, 1H), 1.65 (s, 3H), 1.71 (d, 1H), 1.59 (d, 1H);
¹³C-NMR (CDCl₃) : δ 146.7 (s) 143.9 (s) 133.0 (s), 131.8 (d), 129.4 (d), 126.4 (d), 121.5 (d), 110.9 (d), 88.4 (d), 66.8 (2t), 63.0 (d), 57.7 (d), 57.1 (q), 55.8 (t), 54.1 (2t), 52.1 (s), 48.3 (t), 47.9 (t), 33.5 (t), 32.4 (t);

### Beispiel 18

### (4aS,6S,8aS)-4a,5,9,10,11,12-Hexahydro-3-methoxy-11-propargyl-6H-[1] benzofuro[3a,3,2-ef][2]benzazepin-6-ol (Ia Y₁=H, Y₂=OH, X=H, Z₁=C₃H₃)

2,6 g (-)-Epi-Norgalanthamin (Ia Y₁=H, Y₂=OH, X=H, Z₁=H), 6,1 g Kaliumcarbonat, 3,64 g Kaliumjodid und 1,47 ml 3-Brom-1-propin werden in 150 ml Acetonitril bei Rückflusstemperatur gerührt. Nach 12 Stunden wird die Reaktionsmischung auf Raumtemperatur abgekühlt, das Lösungsmittel unter Vakuum abdestilliert, und der Rückstand mit 300 ml 2N HCl und mit 100 ml Ethylacetat versetzt. Nach dem Abtrennen wird die organische Phase entsorgt. Die wässrige Phase wird mit Ammoniak-Lösung basisch eingestellt und mit 3x100 ml Methylenchlorid extrahiert. Die vereinigten organischen Phasen werden über Natriumsulfat getrocknet, filtriert und unter Vakuum eingeengt. Das Produkt wird säulenchromatographisch (Chloroform:MeOH=95:5) gereinigt.
Ausbeute: 0,8 g (26,1% d. Th)
Smp: 157-160 °c
R_{f}: 0,45 (Chloroform:MeOH=95:5)
¹H-NMR (CDCl₃): δ 6.65 (d, 1H), 6.58 (d, 1H), 6.08 (d, 1H), 5.85 (d, 1H), 4.72 (m, 1H), 4.65 (b, 1H), 4.12 (d, 1H), 3.87 (s, 3H), 3.79 (d, 1H), 3,38 (s, 2H), 3.31 (m, 1H), 3.20 (d, 1H), 2.45 (b, 1H), 2.31 (s, 1H), 2.10 (dt, 1H), 1.72 (m, 2H);
¹³C-NMR (CDCl₃) : δ 146.7 (s) 143.9 (s) 132.9 (s), 131.9 (d), 128.5 (s), 126.4 (d), 121.6 (d), 111.0 (d), 88.4 (d), 79.5 (s), 72.9 (t), 63.0 (d), 58.0 (t), 55.9 (q), 51.7 (t), 48.0 (t), 43.9 (s), 34.9 (t), 32.3 (t);

### Beispiel 19

### (4aS,6S,8aS)-4a,5,9,10,11,12-Hexahydro-3-methoxy-11-benzoyl-6H-[1]benzofuro[3a,3,2-ef][2]benzazepin-6-ol (Ia Y₁=H, Y₂=OH, X=H, Z₁=C₇H₅O)

2,0 g (-)-Epi-Norgalanthamin (Ia Y₁=H, Y₂=OH, X=H, Z₁=H), 3,0 g Kaliumcarbonat und 0,9 ml Benzoylchlorid werden in 50 ml Acetonitril bei Rückflusstemperatur gerührt. Nach einer Stunde wird die Reaktionsmischung auf Raumtemperatur abgekühlt, das Lösungsmittel im Vakuum abdestilliert und der Rückstand mit 100 ml Wasser und 50 ml Ethylacetat versetzt. Nach dem Abtrennen der organischen Phase wird die wässrige Phase mit 2x50 ml Ethylacetat extrahiert. Die vereinigten organischen Phasen werden mit 2x40 ml 1N HCl und 1x20 ml gesättigte NaCl-Lösung (brine) gewaschen, über Natriumsulfat getrocknet, filtriert und im Vakuum eingeengt. Der Rückstand wird aus 2-Butanon kristallisiert.
Ausbeute: 1,5 g (54 % d. Th)
Smp: 198-199 °C
R_{f}: 0,4 (Chloroform:MeOH:Ammoniak-Lösung=95:4,5:0,5)
¹H-NMR (DMSO): δ 7.61 (m, 4H), 7,18 (d, 1H), 6.69 (m, 2H), 6.12 (d, 1H), 5.78 (b, 1H) 4.61 (b, 2H), 4.28 (b, 2H), 3.71 (s, 3H), 3.53 (m, 1H), 3.52 (m, 2H), 1.92 (m, 2H), 1,63 (m, 2H);
1³C-NMR (DMSO): δ170.2 (s) 147.1 (s) 143. 5 (s), 136.6 (s), 132.9 (s), 132.7 (d), 128.7 (s), 128.0 (d), 126.2 (d), 126.0 (d), 125.8 (d), 125.5 (d), 120.8 (d), 119.3 (d), 111.3 (d), 87.4 (d), 61.1 (d), 55.4 (q), 53.1 (t), 48.1 (s), 47.3 (t), 43.1 (t), 31.8 (t);

### Beispiel 20

### (4aS,6S,8aS)-6-Hydroxy-3-methoxy-4a,5,9,10-tetrahydro-6H-[1]benzofuro[3a,3,2-ef][2]benzazepin-11(12H)-thiocarbonic acid allylamide (Ia Y₁=H, Y₂=OH, X=H, Z₁=C₇H₅O)

1,5 g (-)-Epi-Norgalanthamin (Ia Y₁=H, Y₂=OH, X=H, Z₁=H) und 0,6 ml Allylisothiocyanat werden in 50 ml Tetrahydrofuran bei Rückflusstemperatur gerührt. Nach 3 Stunden wird die Reaktionsmischung auf Raumtemperatur abgekühlt und das Lösungsmittel unter Vakuum abdestilliert. Der Rückstand wird säulenchromatographisch (Chloroform:MeOH=97:3) gereinigt.
Ausbeute: 1,9 g (92 % d. Th) weisser Schaum
R_{f}: 0,25 (Chloroform:MeOH=97:3).
¹H-NMR (CDCl₃): δ 6.67 (m, 2H), 6.01 (d, 1H), 5.88 (m, 2H), 5.50 (b, 1H), 5.31 (d, 1H), 5.09 (t, 2H), 4.02 (d, 1H), 4.61 (m, 2H), 4.23 (m, 2H), 3.85 (s, 3H, 3,60 (t, 1H), 2.78 (d, 1H), 2.22 (t, 1H), 1.88 (d, 1H), 1.75 (t, 1H);
ATP-NMR (DMSO): δ 181.6 (s), 148.7 (s) 145.3 (s) 134.2 (d), 132.8 (d), 126.8 (s), 126.4 (d), 1120.3 (d), 117.2 (s), 111.5 (d), 88.7 (d), 63.4 (d), 56.4 (q), 54.0 (t), 51.4 (s), 48.9 (t), 48.7 (t), 36.7 (t), 32.4 (t);

### Beispiel 21

### (4aS,6S,8aS)-4a,5,9,10-Tetrahydro-6-hydroxy-3-methoxy-6H-[1]benzofuro[3a,3,2-ef][2]benzazepin-11(12H)-carboxamid (Ia Y₁=H, Y₂=OH, X=H, Z₁=CH₂NO)

2,2 g (-)-Epi-Norgalanthamin (Ia Y₁=H, Y₂=OH, X=H, Z₁=H) und 1,05 g Natriumcyanid werden in 112 ml Wasser bei Raumtemperatur gerührt und mit 16 ml 2N HCl portionsweise versetzt. Nach 24 Stunden wird der ausgefallene Niederschlag abfiltriert, mit 2x2 ml Wasser nachgewaschen und im Vakuumtrockenschrank 18 Stunden bei 50°C getrocknet.
Ausbeute: 0,56 g (22 % d. Th)
Smp: 168-173 °C
R_{f}: 0,25 (Chloroform:MeOH=9:1)
¹H-NMR (DMSO): δ 6.62 (d, 1H), 6.49 (d, 1H), 6.00 (d, 1H), 5.58 (d, 1H), 4.47 (b, 1H), 4.26 (t, 1H), 3.85 (d, 1H), 3.72 (d, 1H), 3.70 (s, 3H), 3.09 (m, 2H), 2.45 (m, 2H), 1.75 (m, 1H), 1.59 (t, (1H);
¹³C-NMR (DMSO): δ147.6 (s) 143.8 (s) 135.1 (s), 134.2 (s), 133.3 (d), 127.2 (d), 120.3 (d), 112.0 (d), 88.7 (d), 62.2 (d), 56.4 (q), 53.8 (t), 49.0 (s), 47.4 (t), 41.1 (t), 32.9 (t);

### Beispiel 22

### (4aS,6S,8aS)-4a,5,9,10,11,12-Hexahydro-3-methoxy-11-(3-Methylbut-2-en-1-yl)-6H-[1]benzofuro[3a,3,2-ef][2]benzazepin-6-ol (Ia Y₁=H, Y₂=OH, X=H, Z₁=C₅H₉)

2,0 g (-)-Epi-Norgalanthamin (Ia Y₁=H, Y₂=OH, X=H, Z₁=H), 2,02 g Kaliumcarbonat und 1,0 ml 3,3-Dimethylallylbromid werden in 80 ml Acetonitril bei Rückflusstemperatur gerührt. Nach 48 Stunden wird die Reaktionsmischung auf Raumtemperatur abgekühlt, das Lösungsmittel im Vakuum abdestilliert und der Rückstand mit 200 ml 2N HCl und 50 ml Ethylacetat versetzt. Nach dem Abtrennen wird die organische Phase entsorgt. Die wässrige Phase wird mit Ammoniak-Lösung basisch eingestellt und mit 3x100 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=9:1) gereinigt.
Ausbeute: 1,93 g (77 % d. Th)
Smp: 36-48 °C
R_{f}: 0,2 (Chloroform:MeOH:Ammoniak-Lösung=90:9:1)
¹H-NMR (DMSO): δ 6.63 (d, 1H), 6.48 (d, 1H), 6.91 (d, 1H), 5.67 (d, 1H), 3.25 (m, 1H), 3.04 (d, 1H), 4.49 (s, 1H), 4.24 (b, 1H), 3.99 (d, 1H), 3.71 (s, 3H), 3.58 (d, 1H), 3.21 (t, 1H), 3.10 (m, 2H), 2.48 (m, 2H), 2.01 (dt, 1H), 1,68 (s, 3H), 1.62 (dt, 1H 1.43 (s, 3H);
¹³C-NMR (DMSO): δ 147.1 (s), 144.0 (s) 134.4 (s), 133.5 (d), 130.6 (s), 126.7 (d), 123.2 (d), 121.1 (d), 112.1 (d), 88.7 (d), 62.2 (d), 57.8 (s), 56.3 (q), 51.8 (t), 50.4 (s), 48.6 (t), 41.0 (t), 40.0 (t), 39.7 (t) 26.6 (q), 18.8 (q);

### Beispiel 23

### (4aS,6S,8aS)-1-Bromo-11-(4-brombenzyl)-4a,5,9,10,11,12-Hexahydro-3-methoxy-6H-[1]benzofuro[3a,3,2-ef][2]benzazepin-6-ol (Ia Y₁=H, Y₂=OH_{,} X=H, Z₁=C₇H₄Br)

2,0 g (-)-Epi-Norgalanthamin (Ia Y₁=H, Y₂=OH, X=H, Z₁=H), 6,0 g Kaliumcarbonat und 1,92 g 4-Brombenzylbromid werden in 40 ml Tetrahydrofuran bei Raumtemperatur gerührt. Nach 12 Stunden wird die Reaktionsmischung auf Raumtemperatur abgekühlt, der Niederschlag abfiltriert und unter Vakuum eingeengt. Das Produkt wird säulenchromatographisch (Chloroform:MeOH=95:5) gereinigt.
Ausbeute: 1,81 g (56 % d. Th)
Smp: 77-100 °C
R_{f}: 0,3 (chloroform:MeOH:Ammoniak-Lösung=90:9:1)
¹H-NMR (DMSO): δ 7.47 (d, 2H), 7.21 (d, 2H), 6.68 (d, 1H), 6.39 (d, 1H), 6.08 (d, 1H), 5.69 (d, 1H), 3.98 (d, 1H), 4.56 (b, 1H), 4.11 (d, 1H), 3.71 (s, 3H), 4.50 (m, 3H), 3.32 (m, 1H), 2.95 (d, 1H), 2.49 (m, 1H), 2.13 (t, 1H), 1.62 (t, 1H), 1.48 (d, 1H) ;
¹³C-NMR (DMSO): δ 147.3 (s), 144.2 (s), 139.6 (s) 134.2 (s), 133.6 (d), 131.9 (2d), 131.6 (2d), 130.2 (s), 126.6 (d), 121.8 (d), 120.6 (s), 112.2 (d), 88.6 (d), 62.2 (d), 57.6 (t), 55.3 (q), 51.6 (t), 48.7 (s), 41.0 (t), 34.0 (t), 33.0 (t);

### Beispiel 24

### (4aR,6R,8R)-4a,5,9,10,11,12-Hexahydro-3-methoxy-6H-[1]benzofuro[3a,3,2-ef][2]benzazepin-6-ol (Ib Y₁=H, Y₂=OH, X=H, Z₁=H)

10 g (+)-Norgalanthamin , welches gemäß der WO-A-01/74820 hergestellt wurde, werden in 400 ml 2 %-iger HCI-Lösung unter Rückflusskühlung bei Siedetemperatur gerührt. Nach 3 Stunden wird die Reaktionsmischung abgekühlt, mit cc Ammoniak-Lösung basisch eingestellt und der ausgefallene Niederschlag abgetrennt.
Ausbeute: 7,6 g (76 % d. Th)
Smp: 166-168 °C
R_{f}: 0,2 (Chloroform:MeOH:Ammoniak-Lösung=90:9:1)
¹H-NMR (CDCl₃): δ 6.70. (d, 1H), 6.62 (d, 1H), 6.00 (d, 1H), 5.78 (d, 1H), 4.68 (b, 2H), 3.95 (d, 1H), 3.85 (d, 1H), 3.79 (s, 3H), 3.32 (d, 1H), 3.20 (t, 1H), 2.75 (m, 1H), 1.90 (d, 1H), 1.82 (dt, 1H), 1.59 (dt, 1H) ;
¹³C-NMR (CDCl₃): δ 147.5 (s) 144.2 (s) 133.5 (s), 133.4 (s), 132.5 (d), 126.9 (d), 120.5 (d), 111.2 (d), 88.9 (d), 63.0 (d), 56.3 (q), 54.1 (t), 49.0 (s), 47.6 (t), 41.4 (t), 32.7 (t);

### Beispiel 25

### (4aR,6R,8aR)-4a,5,9,10-Tetrahydro-6-hydroxy-3-methoxy-6H-[1]benzofuro[3a,3,2-ef][2]benzazepin-11(12H)-carboxamid (Ib Y₁=H, Y₂=OH, X=H, Z₁=CH₂NO)

2,2 g (+)-Epi-norgalanthamin (Ib Y₁=H, Y₂=OH, X=H, Z₁=H) werden in 112 ml bidestilliertem Wasser gelöst, mit 2N HCI auf pH=3 eingestellt, 1,05g NaOCN zugegeben, und der pH-Wert neuerlich mit 2N HCl auf 3 eingestellt. Das Reaktionsgemisch wird 20 Stunden bei Raumtemperatur gerührt, der entstandene Niederschlag abfiltriert und bei 50 mbar und 60°C im Vakuumtrockenschrank 20 Stunden lang getrocknet. Die erhaltenen 2,2g an Rohprodukt werden in 15 ml MeOH durch Erhitzen auf Rückfluss gelöst, eine Stunde im Eisbad gerührt und filtriert.
Ausbeute: 1, 1g (43,2 % d. Th.) weiße Kristalle
FP: 208-214°C
R_{f}.: 0,45 (Chloroform:MeOH:Ammoniak-Lösung=90:9:1)
¹H-NMR (DMSO): δ 6.72 (d, 1H), 6.68 (d, 1H), 6.08 (d, 1H), 5..87 (b, 2H, NH2), 5.69 (d, 1H), 5.00 (d, 1H), 4.59 (d, 1H), 4.48 (s, 1H), 4.28 (m, 1H), 4.11 (m, 1H), 3.71 (s, 3H), 3.38 (m, 2H), 2.49 (m, 2H), 1.88 (dt, 1H), 1.62 (m, 2H);
¹³C-NMR (DMSO) : δ 158.3 (s), 147.8 (s), 144.2 (s) 133.6 (d), 133.5 (s), 131.1 (s), 126.8 (d), 121.3 (d), 112.0 (d), 88.5 (d), 62.2 (d), 56.4 (q), 48.7 (t), 45.5 (s), 41.0 (t), 37.9 (t), 32.8 (t);

### Beispiel 26

### (4aR,6R,8aR)-4a,5,9,10,11,12-Hexahydro-3-methoxy-11-ethyl-6H-[1]benzofuro[3a,3,2-ef][2]benzazepin-6-ol (Ib Y₁=H, Y₂=OH, X=H, Z₁=C₂H₅)

1,35 g (+) -Epi-Norgalanthamin (Ib Y₁=H, Y₂=OH, X=H, Z₁=H), 2,0 g Kaliumcarbonat und 0,8 ml Ethylbromid werden in 50 ml Tetrahydrofuran bei Rückflusstemperatur gerührt. Nach 70 Stunden wird die Reaktionsmischung auf Raumtemperatur abgekühlt, das Lösungsmittel im Vakuum abdestilliert und der Rückstand mit 50 ml Wasser und mit 50 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH:Ammoniak-Lösung=95:4,5:0,5) gereinigt.
Ausbeute: 1,0 g (67,2 % d. Th)
Smp: 135-136 °C
R_{f}: 0,2 (Chloroform:MeOH:Ammoniak-Lösung=95:4,5:0,5)
¹H-NMR (DMSO): δ 6.63 (d, 1H), 6.50 (d, 1H), 6.05 (d, 1H), 5.68 (d, 1H), 4.95 (b, 1H), 4.48 (s, 1H), 4.27 (b, 1H), 4.02 (d, 1H), 3.72 (s, 3H), 3.68 (d, 1H), 3.29 (t, 1H), 3.08 (d, 1H), 2.41 (m, 2H), 2.03 (t, 1H), 1.62 (t, 1H), 1.57 (d, 1H), 0.91 (t, 3H);
¹³C-NMR (DMSO): δ 147.2 (s) 144.0 (s) 134.1 (s), 133.5 (d), 130.3 (s), 126.7 (d), 121.8 (d), 112.1 (d), 88.6 (d), 62.2 (d), 56.9 (d), 56.3 (q), 51.7 (t), 48.7 (s), 45.5 (t), 33.7 (t), 33.0 (t), 13.5 (q);

### Beispiel 27

### Methyl(4aR,6R,8aR)-N11-cyano-6-hydroxy-3-methoxy-4a,5,9,10-tetrahydro-6H-[1]benzofuro[3a,3,2-ef][2]benzazepin-11(12H)-carboximidothioat (Ib Y₁=H, Y₂=OH, X=H, Z₁=C₂H₅)

2,5 g (+) -Epi-Norgalanthamin (Ib Y₁=H, Y₂=OH, X=H, Z₁=H) und 1,05 g Dimetyl-N-cyandithioiminocarbonat werden in 80 ml Ethanol und 20 ml Dimethylformamid bei Rückflusstemperatur gerührt. Nach 21 Stunden wird die Reaktionsmischung auf Raumtemperatur abgekühlt und das Lösungsmittel im Vakuum abdestilliert. Der Rückstand wird säulenchromatographisch (Chloroform:MeOH=98:2) gereinigt.
Ausbeute: 0,72 g (20,5 % d. Th)
Smp: 78-79 °C
R_{f}: 0,35 (Chloroform:MeOH:Ammoniak-Lösung=90:9:1)
¹H-NMR (DMSO): δ 6.72 (m, 2H), 6.12 (d, 1H), 5.72 (d, 1H), 5.05 (m, 2H), 6.19 (m, 2H), 4.26 (b, 1H), 3.72 (s, 3H), 3.32 (b, 1H), 2.62 (s, 3H), 2.49 (m, 1H), 1.89 (m, 2H), 1.65 (m, 1H);
¹³C-NMR (DMSO): δ 148.0 (s), 144.9 (s) 134.1 (d), 133.0 (s), 127.3 (s), 126.3 (d), 121.7 (d), 115.5 (s), 115.0 (s), 112.3 (d), 88.2 (d), 62.0 (d), 56.4 (q), 48.4 (t), 41.0 (s), 40.9 (t), 40.8 (t), 32.7 (t), 16.6(q);

### Beispiel 28

### (4aR,6R,8aR)-4a,5,9,10,11,12-Hexahydro-3-methoxy-11-methyl-6H-benzofuro[3a,3,2-ef][2]benzazepin-6-amin, Dihydrochloride

3,0 g (+)-Galanthamin, hergestellt nach Kametani, Heterocycles 4, 1111, 1976, sowie 3,3 g Triphenylphosphin und 18 ml Stickstoffwasserstoffsäure (1,06 mol/lit in Benzol) werden in 225 ml Tetrahydrofuran aufgelöst, bei Raumtemperatur mit 7,0 ml Azodicarbonsäurediethylester (40 % in Toluol) versetzt und intensiv gerührt. Nach 20 Stunden wird die Reaktionsmischung mit 150 ml 2N HCl versetzt, eine Stunden intensiv gerührt, die organische Phase abgetrennt, und die wässrige Phase mit 2x50 ml Ethylacetat gewaschen. Der pH-Wert der wässrigen Phase wird mit Ammoniak-Lösung auf 12 gestellt und die trübe Suspension mit 3x80 ml Methylenchlorid extrahiert. Die vereinigten organischen Phasen werden über Natriumsulfat getrocknet, filtriert und unter Vakuum eingeengt. Das Produkt wird säulenchromatographisch (Chloroform:MeOH:Ammoniak-Lösung=95:4,5:0,5) gereinigt. Das erhaltene Öl wird in Isopropanol aufgelöst und das Hydrochlorid-Salz aus etherischer HCI gefällt.
Ausbeute: 1,54 g (41 % d. Th)
Smp: 235-250 °c
R_{f}: 0,45 freie Base (Chloroform:MeOH:Ammoniak-Lösung=95:4,5:0,5)
¹H-NMR (CDCl₃): δ 6.69 (d, 1H), 6.60 (d, 1H), 6.21 (d, 1H), 5.75 (d, 1H), 4.62 (b, 1H), 4.37 (m, 1H), 4.08 (d, 1H), 3.89 (s, 3H), 3.67 (d, 1H), 3.29 (t, 1H), 3.09 (d, 1H), 2.81 (m, 1H), 2.41 (s, 3H), 2.22 (dt, 1H), 1.85 (dt, 1H), 1.67 (d, 1H);
¹³ C-NMR (CDCl₃) : δ 146.9 (s) 144.3 (s) 132.8 (s), 1.29. 8 (s), 129.3 (d), 126.9 (d), 122.1 (d), 111.5 (d), 87.9 (d), 60.8 (d), 56.3 (q), 54.4 (t), 53.8 (q), 48.6 (s), 42.5 (d), 34.7 (t), 29.1 (t);

### Beispiel 29

### (4aR,6S,8aR)-11-(3-hydroxypropyl)-3-methoxy-5,6,9,10,11,12-hexahydro-4aH-[1]benzofuro[3a,3,2-ef][2]benzazepin-6-ol (Ib Y₁=OH, Y₂=H, X=H, Z₁=C₃H₇O)

1,3 g (+)-Norgalanthamin, welches gemäß der WO-A-01/74820 hergestellt wurde, sowie 2,6 g Kaliumcarbonat und 0,65 ml 3-Brom-1-propanol werden in 70 ml Acetonitril bei Rückflusstemperatur gerührt. Nach 48 Stunden wird die Reaktionsmischung auf Raumtemperatur abgekühlt, das Lösungsmittel unter Vakuum abdestilliert und der Rückstand mit 90 ml Wasser und mit 40 ml Chloroform versetzt. Nach dem Abtrennen der organischen Phase wird die wässrige Phase mit 2x30 ml Chloroform extrahiert. Die vereinigten organischen Phasen werden über Natriumsulfat getrocknet, filtriert und unter Vakuum eingeengt. Das Produkt wird säulenchromatographisch (Chloroform:MeOH=9:1) gereinigt.
Ausbeute: 0,86 g (54,5 % d. Th) harziges Öl
R_{f}: 0,45 (Chloroform:MeOH=9:1)
¹H-NMR (CDCl₃) : δ 6.66 (m, 2H), 6.05 (m, 2H), 4.59 (b, 1H), 4.14 (m, 1H), 4.09 (d, 1H), 3.95 (d, 1H), 3.81 (s, 3H), 3.79 (t, 2H), 3.31 (m, 2H), 2.75 (m, 3H), 2.05 (m, 2H), 1.78 (m, 1H), 1.59 (m, 2H);
¹³C-NMR (CDCl₃) : δ 146.2 (s) 144.7 (s) 133.5 (s), 128.7 (s), 128.2 (d), 127.0 (d), 122.7 (d), 111.6 (d), 89.1 (d), 64.8 (t), 62.4 (d), 57.8 (d), 56.3 (q), 52.4 (t), 52.1 (t), 48.7 (s), 33.3 (t), 30.3 (t), 27.9 (t);

### Beispiel 30

### (4aS,6R,8aS)-6-hydroxy-3-methoxy-5,6,9,10-tetrahydro-4aH-[1]benzofuro[3a,3,2-ef][2]benzazepine-11(12H)-carbothioamide (Ib Y₁=OH, Y₂=H, X=H, Z₁=CH₂NO)

2,2 g (+)-Norgalanthamin , welches gemäß der WO-A-01/74820 hergestellt wurde, werden in 112 ml bidestilliertem Wasser gelöst, mit 2N HCl auf pH=3 eingestellt, 1,05g NaOCN zugegeben und der pH-Wert neuerlich mit 2N HCl auf 3 eingestellt. Das Reaktionsgemisch wird 20 Stunden bei Raumtemperatur gerührt, mit konz. Ammoniak-Lösung basisch eingestellt und 3x mit je 40 ml Dichlormethan ausgeschüttelt. Die vereinigten organischen Phasen werden über Natriumsulfat getrocknet, filtriert und eingedampft. Die erhaltenen 2,5 g werden an 170 g Kieselgel mit dem Laufmittel Chloroform:Methanol=95:5 säulenchromatographisch gereinigt, und die gereinigten Fraktionen eingedampft.
Ausbeute: 1,08 g (42,4%d. Th.) weiße Kristalle
FP: 101-114°C
R_{f}.: 0,29 (Chloroform:MeOH=95:5)
¹H-NMR (DMSO): δ 6.75 (d, 1H), 6.69 (d, 1H), 6.08 (d, 1H), 5.87 (b, 2H, NH2), 5.78 (dd, 1H), 5.60 (d, 1H), 4.54 (b, 1H), 4.22 (m, 2H), 4.05 (m, 1H), 3.70 (s, 3H), 3.39 (m, 1H), 2.29 (d, 1H), 2.05 (dd, 1H), 1.78 (t, 1H), 1.60 (d, 1H);
¹³C-NMR (DMSO): δ 158.3 (s), 147.6 (s), 144.2 (s) 133.1 (d), 131.1 (s), 129.0 (d), 127.8 (d), 121.1 (d), 111.9 (d), 87.3 (d), 60.6 (d), 56.4 (q), 51.0 (t), 48.6 (s), 45.5 (t), 38.1 (t), 31.6 (t);

### Beispiel 31

### (4aS,6S,8aS)-4a,5,9,10,11,12-Hexahydro-3-methoxy-11-benzoyl-6H-[1]benzofuro[3a,3,2-ef][2]benzazepin-6-ol

### Stufe 1

Eine Lösung von 6,0 g (+)-Galanthamin, hergestellt nach Kametani, Heterocycles 4, 1111, 1976), in 42 ml Eisessig wird mit einer Mischung von 12,7 ml Salpetersäure und 21 ml Eisessig bei 0-5 °C tropfenweise versetzt. Nach 15 min wird die Reaktionsmischung auf 100 ml Wasser getropft, der pH-Wert mit Ammoniak-Lösung auf 12 gestellt und mit 100 ml Chloroform versetzt. Nach dem Abtrennen der organischen Phase wird die wässrige Phase mit 3x50 ml Chloroform extrahiert. Die vereinigten organischen Phasen werden über Natriumsulfat getrocknet, filtriert und unter Vakuum eingeengt. Das Produkt wird säulenchromatographisch (Chloroform:MeOH=95:5) gereinigt.
Ausbeute: 1,47 g (19,4 % d. Th) braunes Öl
R_{f}: 0,25 (Chloroform:MeOH=95:5)

### Stufe 2

1,47 g des aus Stufe 1 erhaltenen Produktes, 2,94 g Zinkpulver und 1,47 g CaCl₂ werden in 44 ml Ethanol und 22 ml Wasser bei Rückflusstemperatur gerührt. Nach 3 Stunden wird der erhaltene Niederschlag abfiltriert und das Lösungsmittel abdestilliert. Das Produkt wird säulenchromatographisch (Chloroform:MeOH:Ammoniak-Lösung=90:9:1) gereinigt.
Ausbeute: 0,36 g (43,4 % d. Th)
Smp: 166-167 °C
R_{f}: 0,3 (Chloroform:MeOH:Ammoniak-Lösung=90:9:1)
¹H-NMR (DMSO): δ 6.18 (s, 1H), 6.04 (d, 1H), 5.78 (dd, 1H), 4.41 (b, 2H, NH2), 4.33 (b, 1H) , 4.01 (d, 1H), 3.82 (d, 1H), 3.58 (s, 3H), 3.55 (d, 1H), 3.18 (t, 1H), 2.87 (d, 1H), 2.28 (s, 3H), 2.22 (d, 1H), 1.98 (d, 1H), 1.89 (t, 1H), 155 (d, 1H);
¹³C-NMR (DMSO) : δ 158.3 (s), 147.6 (s), 144.2 (s) 133.1 (d), 131.1 (s), 129.0 (d), 127.8 (d), 121.1 (d), 111.9 (d), 87.3 (d), 60.6 (d), 56.4 (q), 51.0 (t), 48.6 (s), 45.5 (t), 38.1 (t), 31.6 (t);

### Beispiel 32

### 2-((4aS,6R,8aS)-6-Hydroxy-3-methoxy-5,6,9,10-tetrahydro-4aH-[1]benzofuro[3a,3,2-ef][2]benzazepin-11(12H)-yl)-1-methyl-1-(3-phenoxyphenyl)-ethane Hydrochloride (Ia Y₁=OH, Y₂=H, X=H, Z₁=C₁₅H₁₅O)

### Stufe 1

3,63 g (+/-)-2-[3-Phenoxyphenyl]-1-propansäure werden in 20 ml Tetrahydrofuran gelöst, 700 mg Lithiumaluminiumhydrid zugegeben und 1 Stunde bei Raumtemperatur gerührt. Danach werden vorsichtig 50 ml Wasser zugetropft, 1 Stunde gerührt und filtriert. Das klare Filtrat wird 3mal mit je 20 ml Ethylacetat extrahiert, die vereinigten organischen Phasen mit Natriumsulfat getrocknet, filtriert und das Lösungsmittel abgezogen.
Ausbeute: 2,4, g (70 % d. Th.) farbloses Öl
R_{f}.. 0,36 (Petrolether:Ethylacetat=4:1)

### Stufe 2

3,15 g Triphenylphosphin werden in 90ml Tetrahydrofuran gelöst, 0,6ml Brom zugetropft und zur entstandenen Suspension 2,4 g (+/-)-2-[3-Phenoxyphenyl]-1-propanol aus Stufe 1 in festem Zustand zugegeben. Nach 30 Minuten werden 100 ml Wasser zugegeben, 3mal mit je 30 ml Ethylacetat extrahiert, die organischen Phasen vereinigt, über Natriumsulfat getrocknet, filtriert und das klare Filtrat durch eine kurze Kieselgelsäule gesaugt. Nach Abdampfen des Lösungsmittels werden 2,7 g (88 % d. Th.) eines farblosen Öles erhalten. Das entstandene (+/-) -2- [3-Phenoxyphenyl]-1-Brompropan wurde sofort für die nächste Stufe verwendet.

### Stufe 3

4,56 g (-)-Norgalanthamin HCl, welches gemäß der WO-A-01/74820 hergestellt wurde, sowie 4g 3-(1-Brom-2-Propyl)-diphenylether und 9,97g Kaliumcarbonat werden in 53 ml Acetonitril bei 85°C 40 Stunden lang gerührt. Die Suspension wird in 10 ml Wasser gegossen und 3mal mit je 30 ml Ethylacetat extrahiert. Nach dem Trocknen der organischen Phase über Natriumsulfat wird diese filtriert und eingedampft und die erhaltenen 4,5 g an 400 g Kieselgel mit Ethylacetat säulenchromatographisch gereinigt. Die produkthaltigen Fraktionen werden vom Lösungsmittel befreit, in 50 ml Diethylether aufgenommen, und das Hydrochlorid mit etherischer HCl ausgefällt.
Ausbeute: 1,5 g (19 % d. Th.)
Smp: 109-115°C
R_{f}.: 0,67 (Ethylacetat)
¹H-NMR (CDCl₃): δ 7.35 (m, 2H), 7.24 (m, 1H), 7.13 (m, 1H), 7.00 (m, 2H), 6.89 (m, 3H), 6.62(m, 2H), 6.04 (m, 2H), 4.60 (b, 1H), 4.14 (m, 2H), 3.85 (s, 3H), 3.77 (t, 1H), 3.38 (m, 1H), 3.12 (m, 1H), 2.91 (m, 1H), 2.70 (m, 2H), 2.48 (m, 1H), 1.99 (m, 2H), 1.48 (m, 1H), 1,25 (m, 3H);
¹³C-NMR (CDCl₃): δ 157.3 (s), 157.2 (s), 157.0 (s), 148.3 (s), 148.2 (s), 145.8 (s) 144.0 (s) 133.2 (s), 133.1 (s), 129.9 (s), 129.6 (d), 129.5 (d), 129.4 (d), 127.5 (d), 127.4 (d), 127.0 (d), 123.0 (d), 122.9 (d), 122.2 (d), 122.1 (d), 121.9 (d), 121.8 (d), 118.7 (d), 118.6 (d), 111.0 (d), 88.7 (d), 62.1 (d), 58.0 (t), 57.0 (t), 55.9 (q), 52.3 (t), 51.2 (t), 48.5 (s), 48.4 (s), 37.9 (d), 37.7 (d), 32.8 (t), 32.5 (t), 29.9 (t), 20.0 (q), 19.5(q);

### Beispiel 33

### (4aS,6R,8aS)-4a,5,9,10,11,12-Hexahydro-3-methoxy-11-benzoyl-6H-[1]benzofuro[3a,3,2-ef][2]benzazepin-6-ol (Ia Y₁=OH, Y₂=H, X=H, Z₁=C₇H₅O)

2,0 g (-)-Norgalanthamin, welches gemäß der WO-A-01/74820 hergestellt wurde, wird in 50 ml Acetonitril unter leichtem Erwärmen aufgelöst und auf 0-10 °C abgekühlt. Die Reaktionsmischung wird mit 3,0 g Kaliumcarbonat und 0,9 ml Benzoylchlorid versetzt und bei Raumtemperatur weitergerührt. Nach einer Stunde wird das Lösungsmittel im Vakuum abdestilliert und der Rückstand mit 50 ml Wasser und 20 ml Ethylacetat versetzt. Nach dem Abtrennen der organischen Phase wird die wässrige Phase mit 3x20 ml Ethylacetat extrahiert. Die vereinigten organischen Phasen werden mit 2x20 ml 1N HCl und 1x20 ml Wasser gewaschen, über Natriumsulfat getrocknet, filtriert und unter Vakuum eingeengt. Der Rückstand wurde aus 2-Butanon und tert.-Butylmethylether (MTBE) kristallisiert.
Ausbeute: 1,76 g (63,7 % d. Th)
Smp: 152-154 °C
R_{f}: 0,75 (Chloroform:MeOH:Ammoniak-Lösung=95:4,5:0,5)
¹H-NMR (DMSO): δ 7.39 (m, 4H), 7.11 (d, 1H), 6.72 (b, 1H), 6.63 (d, 1H), 6.09 (m, 1H), 5.81 (b, 1H), 4.62 (d, 1H), 4.55 (b, 1H), 4.31 (m, 2H), 4.09 (d, 1H), 3.71 (s, 3H), 3.48 (m, 1H), 2.32 (t, 1H), 2.09 (m, 1H), 1.89 (b, 1H), 1.70 (m, 1H);
¹³C-NMR (DMSO): δ171.0 (s) 147.8 (s) 144.5 (s), 137.5 (s), 133.3 (s), 130.1 (d), 129.7 (s), 129.4 (d), 129.3 (d), 129.0 (d), 127.7 (d). 127.5 (d), 121.6 (d), 120.3 (d), 112.3 (d), 87.3 (d), 60.5 (d), 56.4 (q), 54.2 (t), 48.5 (s), 44.0 (t), 37.4 (t), 31.4 (t);

### Beispiel 34

### 2-((4aS, 6R, 8aS) -6-Hydroxy-3-methoxy-5, 6, 9, 10-tetrahydro-4aH-[1]benzofuro[3a,3,2-ef][2]benzazepin-11(12H)-yl)acetamide (Ia Y₁=OH, Y₂=H, X=H, Z₁=C₂H₄NO)

3,0 g (-)-Norgalanthamin HCl, welches gemäß der WO-A-01/74820 hergestellt wurde, sowie 3,0 g Kaliumcarbonat und 1,4 g 2-Bromacetamid werden in 50 ml Acetonitril unter Rückfluss gerührt. Nach 3 Stunden wird der erhaltene Niederschlag warm abfiltriert, das Lösungsmittel im Vakuum entfernt und der Rückstand aus Ethanol auskristallisiert.
Ausbeute: 1,74 g (54,4 % d. Th)
Smp: 107-113 °C
R_{f}: 0,5 (Chloroform:MeOH:Ammoniak-Lösung=89:10:1)
¹H-NMR (DMSO): δ 7.18 (d, 2H, NH2), 6.71 (d, 1H), 6.52 (d, 1H), 6.07 (d, 1H), 5.81 (dd, 1H), 4.48 (b, 1H), 4.27 (d, 1H), 4.19 (d, 1H), 4.05 (b, 1H), 3.71 (s, 3H), 3.63 (d, 1H), 3.28 (t, 1H), 3.00 (d, 1H), 2.88 (d, 1H), 2.27 (d, 1H), 2.06 (m, 1H), 1.93 (t, 1H), 1.46 (d, 1H);
¹³C-NMR (DMSO): δ 173.2 (s), 147.1 (s), 144.3 (s) 133.6 (s), 130.2 (s), 129.2 (d), 127.7 (d), 121.9 (d), 112.1 (d), 87.6 (d), 60.8 (d), 58.5 (t), 56.4 (q), 56.3 (t), 52.6 (s), 48.6 (t), 35.1 (t), 31.7 (t);

### Beispiel 35

### 3-[(4aS,6R,8aS)-6-Hydroxy-3-methoxy-5,6,9,10-tetrahydro-4aH-[1]benzofuro[3a,3,2-ef][2]benzazepin-11(12H)-yl]-2-methyl-1(-4-methyl-phenyl)propan-1-on, Hydrochloride (Ia Y₁=OH, Y₂=H, X=H, Z₁=C₁₁H₁₃O)

6,0 g (-)-Norgalanthamin HCl, welches gemäß der WO-A-017/4820 hergestellt wurde, sowie 3,3 g 4-Methylpropiophenon, 6,0 ml 1,3-Dioxolan und 0,2 ml 2N HCl werden unter Rückfluss gerührt. Nach 3 Stunden wird das Lösungsmittel abdestilliert und der Rückstand mit 100 ml Wasser und mit 50 ml Ethylacetat versetzt. Der pH-Wert der wässrigen Phase wird mit Ammoniak-Lösung auf 9 eingestellt. Nach dem Abtrennen der organischen Phase wird die wässrige Phase mit 3x50 ml Ethylacetat extrahiert. Die vereinigten organischen Phasen werden mit 1x20 ml gesättigter NaCl-Lösung (brine) und 1x20 ml Wasser gewaschen, über Natriumsulfat getrocknet, filtriert und im Vakuum eingeengt und das Produkt durch Säulenchromatographie (Ethylacetat : n Hexan=1:1 zu 8:2) gereinigt. Die erhaltene Substanz wird in Ethylacetat aufgenommen und das Hydrochlorid-Salz mit etherischer HCl gefällt.
Ausbeute: 4,4 g (48 % d. Th)
Smp: 143-151 °C
R_{f}: 0,4 (Chloroform:MeOH=97:3)
¹H-NMR (CDCl₃): δ 7.84 (m, 2H), 7.26 (m, 2H), 6.68 (m, 2H), 6.05 (m, 2H), 4.61 (b, 1H), 4.14 (m, 2H), 3.86 (s, 3H), 3.71 (m, 2H), 3.34 (m, 1H), 3.12 (m, 2H), 2.69 (d, 1H), 2.55 (m, 1H), 2.30 (m, 4H), 2.03 (m, 2H), 1.49 (d, 1H), 1.15 (m, 3H);
¹³C-NMR (CDCl₃) : δ 203.4 (s), 145.9 (s), 144.2 (s) 143.8 (s), 134.4 (s), 133.3 (s), 129.7 (s), 129.3 (d), 129.2 (d), 128.4 (d), 128.3 (d), 127.6 (d), 126.9 (d), 122.0 (d) , 111.1 (d), 88.7 (d), 62.1 (d), 57.8 (t), 55.9 (q), 54.1 (t), 52.2 (t), 48.5 (s), 39.1 (d), 32.9 (t), 29.9 (t), 21,6 (q), 16.5 (q);

### Beispiel 36

### 1-[(4aS,6R,8aS)-6-hydroxy-3-methoxy-5,6,9,10-tetrahydro-4aH-[1]benzofuro[3a,3,2-ef][2]benzazepin-11(12H)-yl]-2-(1-piperidyl) ethane-1-on (Ia Y₁=OH, Y₂=H, X=H, Z₁=C₇H₁₂NO)

3,0 g (-)-Norgalanthamin , welches gemäß der WO-A-017/4820 hergestellt wurde, sowie 1,86 ml Triethylamin und 0,6 ml Chroracetylchlorid werden in 150 ml Tetrahydrofuran bei 0 °C gerührt. Nach 10 min wird die Reaktionsmischung mit 3,0 g Kaliumcarbonat und 0,93 ml Piperidin versetzt und bei 90 °C weitergerührt. Nach 48 Stunden wird das Lösungsmittel abdestilliert, der Rückstand mit 50 ml Wasser und 50 ml Chloroform versetzt. Nach dem Abtrennen der organischen Phase wird die wässrige Phase mit 2x30 ml Chloroform extrahiert. Die vereinigten organischen Phasen werden über Natriumsulfat getrocknet, filtriert und unter Vakuum eingeengt. Das Produkt wird säulenchromatographisch (Chloroform:MeOH=95:5) gereinigt.
Ausbeute: 2,06 g (47,1 % d. Th) harziges Öl
R_{f}: 0,3 (Chloroform:MeOH=:9:1)
¹H-NMR (CDCl₃) : δ 6.69 (m, 2H), 5.98 (m, 2H), 5.19 (d, 1H), 4.56 (b, 1H); 4.37 (d, 1H), 4.15 (d, 1H), 3.80 (s, 3H), 3.27 (d, 1H), 3.18 (m, 1H), 2.89 (d, 1H), 2.69 (d, 1H), 2.41 (m, 5H), 2.06 (d, 1H), 1.91 (m, 1H), 1.75 (d, 1H), 1.50 (m, 4H), 1.39 (b, 1H);
¹³C-NMR (CDCl₃) : δ 196.5 (s), 146.8 (s), 144.6 (s), 132.5 (s), 128.7 (s), 128.0 (s),128.2 (d), 126.5 (d), 122.0 (d), 111.3 (d), 88.4 (d), 62.3 (t), 61.8 (d), 55.9 (d), 55.8 (q), 55.6 (t), 52.2 (s), 45.0 (t), 38.6 (t), 35.7 (t), 29.9 (t), 25.8 (t), 23.9 (t);

### Beispiel 37

### (4aS,6R,8aS)-3-methoxy-11-(2-pyrimidinyl)-5,6,9,10,11,12-hexahydro-4aH-[1]benzofuro[3a,3,2-ef][2]benzazepin-6-ol (Ia Y₁=OH, Y₂=H, X=H, Z₁=C₄H₃N₂)

2,0 g (-)-Norgalanthamin, welches gemäß der WO-A-017/4820 hergestellt wurde, sowie 2,45 g NaHCO₃ und 0,88 ml 2-Chlorpyrimidin werden in 120 ml Ethanol bei Siedetemperatur gerührt. Nach 44 Stunden wird das Lösungsmittel abdestilliert, der Rückstand mit 120 ml Wasser und 100 ml Ethylacetat versetzt. Nach dem Abtrennen der organischen Phase wird die wässrige Phase mit 2x100 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=98:2) gereinigt
Ausbeute: 1,26 g (49 % d. Th)
Smp: 232-235 °C
R_{f}: 0,7 (Chloroform:MeOH:Ammoniak-Lösung=89:10:1)
1H-NMR (DMSO): δ 8.28 (m, 2H), 6.78 (d, 1H), 6.69 (d, 1H), 6.58 (t, 1H), 6.23 (d, 1H), 5.82 (dd, 1H), 5.25(d, 1H), 4.66 (d, 1H), 4.52 (d, 1H), 4.30 (b, 1H), 4.11 (b, 1H), 3.80 (s, 3H), 3.73 (m, 1H), 2.28 (d, 1H), 2.02 (d, 1H), 1.81 (t, 1H), 1.72 (d, 1H);
¹³C-NMR (DMSO): δ 161.2 (s), 158.7 (d), 147.6 (s), 144.1 (s) 133.3 (s), 130.6 (s), 129.0 (2d), 128.0 (d), 121.9 (d), 112.0 (d), 110.7 (d), 87.2 (d), 60.6 (d), 56.4 (q), 51.3 (t), 48.7 (s), 45.5 (t), 36.6 (t), 31.5 (t);

### Beispiel 38

### 1-[(4aS,6R,8aS)-6-Hydroxy-3-methoxy-5,6,9,10-tetrahydro-4aH-[1]benzofuro[3a,3,2-ef][2]benzazepin-11(12H)-yl]-3-(1-pyrrolidyl)propan-1-on (Ia Y₁=OH, Y₂=H, X=H, Z₁=C₇H₁₂NO)

2,0 g (-)-Norgalanthamin, welches gemäß der WO-A-017/4820 hergestellt wurde, sowie 0,86 ml Triethylamin und 0,76 ml 3-Brompropionsäurechrorid werden in 130 ml Aceton bei Raumtemperatur gerührt. Nach 60 min wird das Lösungsmittel abdestilliert und der Rückstand mit 100 ml 2N HCl-Lösung und 50 ml Ethylacetat versetzt. Nach dem Abtrennen der organischen Phase wird die wässrige Mutterlauge mit 2x50 ml Ethylacetat extrahiert. Die vereinigten organischen Phasen werden über Natriumsulfat getrocknet, filtriert und unter Vakuum eingeengt. Der ölige Rückstand wird in 50 ml Acetonitril aufgenommen, mit 5 ml Pyrrolidin versetzt und bei Siedetemperatur gerührt. Nach 8 Stunden wird das Reaktionsgemisch abgekühlt, das Lösungsmittel im Vakuum entfernt, und der Rückstand mit 30 ml 25 %-iger Ammoniak-Lösung und 30 ml Ethylacetat versetzt. Nach dem Abtrennen der organischen Phase wird die wässrige Mutterlauge mit 2x30 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH:Ammoniak-Lösung=89:10:1) gereinigt.
Ausbeute: 1,4 g (48,0 % d. Th)
Smp: 56-63°C
R_{f}: 0,25 (Chloroform:MeOH:Ammoniak-Lösung=89:10:1)
¹H-NMR (DMSO): δ 6.81 (b, 2H), 6.71 (d, 1H), 6.65 (d, 1H), 6.15 (d, 1H), 5.79 (dd, 1H), 4.68 (d, 1H), 4.60 (d, 1H), 4.45 (m, 2H), 4.08 (b, 1H), 3.75 (s, 3H), 3.43 (b, 1H), 2.25 (m, 1H), 2.70 (m, 1H), 2.59 (m, 1H), 2.38 (m, 6H), 2.06 (d, 1H), 1.82 (t, 1H), 1.62 (m, 4H);
¹³C-NMR (DMSO): δ 171.3 (s), 147.9 (s), 144.6 (s) 133.0 (s), 129.7 (s), 129.2 (d), 127.7 (d), 121.5 (d), 112.1 (d), 87.4 (d), 60.6 (d), 56.4 (q), 52.3 (2t), 48.6 (s), 46.5 (t), 44.5 (t), 39.2 (t), 37.2 (t), 32.8 (t), 31.5 (t), 23.9 (2t);

### Beispiel 39

### ((4aS,6R,8aS)-4a,5,9,10,11,12-Hexahydro-6-hydroxy-3-methoxy-6H-benzofuro[3a,3,2-ef][2]benzazepin-11-yl)gamma-oxo-butyric acid (Ia Y₁=OH, Y₂=H, X=H, Z₁=C₄H₅O₃)

2,0 g (-)-Norgalanthamin, welches gemäß der WO-A-017/4820 hergestellt wurde, sowie 1,53 ml Triethylamin und 0,76 g Bersteinsäureanhydrid werden in 70 ml Tetrahydrofuran bei Siede-Temperatur gerührt. Nach einer Stunde wird das Lösungsmittel abdestilliert, der Rückstand mit 100 ml 1N HCl und 100 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und unter Vakuum eingeengt.
Ausbeute: 1,4 g (51,28 % d. Th)
Smp: 156-158 °C
R_{f}: 0,7 (Ethylacetat:Ameisensäure=99:1)
¹H-NMR (DMSO): δ 11.95 (b, 1H, OH), 6.81 (b, 1H), 6.71 (dd, 1H), 6.12 (d, 1H), 5.85 (dd, 1H), 4.68 (d, 1H), 4.60 (d, 1H), 4.43 (m, 2H), 4.09 (b, 1H), 3.71 (s, 3H), 2.25 (t, 1H), 2.89 (m, 1H), 2.35 (m, 3H), 2.09 (m, 2H), 1.80 (b, 1H), 1.65 (m, 1H);
¹³C-NMR (DMSO): δ 174.8 (s), 171.2 (s), 147.9 (s), 144.6 (s) 133.0 (s), 129.5 (s), 129.2 (d), 127.7 (d), 121.4 (d), 112.1 (d), 87.3 (d), 60.6 (d), 56.3 (q), 51.9 (t), 48.6 (s), 46.4 (t), 39.7 (t), 31.5 (t), 29.8 (t), 28.5 (t);

### Beispiel 40

### 1-((4aS,6R,8aS)-6-Hydroxy-3-methoxy-5,6,9,10-tetrahydro-4aH-[1]benzofuro[3a,3,2-ef][2]benzazepin-11(12H)-yl)-2-[2-(2,6-dichloranilino)]-phenylethane (Ia Y₁=OH, Y₂=H, X=H, Z₁=C₁₁H₁₃O)

### Stufe 1

6,75 g o-(2,6)-Dichloranilino)-phenethylalkohol, welches gemäß der DE -A-2007700 hergestellt wurde, werden zu einer Suspension von 7,12 g Triphenylphosphin und 1,36 ml Brom in 100 ml Tetrahydofuran bei Raumtemperatur portionsweise zugegeben. Nach einer halben Stunde werden 100 ml Wasser zugesetzt, 3mal mit je 25 ml Ethylacetat extrahiert, die organische Phase über Natriumsulfat getrocknet, filtriert und bis auf 40 ml eingeengt. Nach Zugabe von 100 ml n-Hexan und Rühren im Eisbad für eine halbe Stunde wird das ausgefallene Triphenylphosphinoxid abfiltriert. Das klare Filtrat wird über eine kurze Kieselgelsäule filtriert und das Lösungsmittel abdestilliert. Es verblieben 8,2 g eines gelblichen Öles, das ohne weitere Reinigung direkt in die nächste Stufe eingesetzt wurde.

### Stufe 2

2,16 g (-)-Norgalanthamin HCl, welches gemäß der WO-A-01/74820 hergestellt wurde, sowie 2,7g (2,6-Dichlorphenyl)-2-(2-Bromethyl)-phenylamin und 4,72 g Kaliumcarbonat werden in 25 ml Acetonitril bei Raumtemperatur 24 Stunden lang gerührt. Danach wird die Suspension in 100 ml Wasser gegossen und 3mal mit je 30 ml Ethylacetat extrahiert. Nach dem Trocknen der organischen Phase über Natriumsulfat und anschließendem Filtrieren wird das Lösungsmittel abdestilliert. Die erhaltenen 4 g werden an 200 g Kieselgel mit Ethylacetat säulenchromatographisch gereinigt, und die produkthältigen Fraktionen eingedampft. Eine Fällung des Hydrochloridsalzes mit etherischer HCl der verbliebenen 800 mg in 15 ml Diethylether bei 0°C liefern 830 mg, die aus 30 ml Ethanol umkristallisiert wurden.
Ausbeute: 700 mg (16 % d. Th.) weiße Kristalle
Smp: 163-165 °C
R_{f}.: 0,5 (Chloroform: MeOH= 9:1)
¹H-NMR (CDCl₃): δ 7.42 (b, 1H, NH), 7.35 (d, 2H), 7.13 (dd, 1H), 7.05 (td, 1H), 7.01 (t, 1H), 6.88 (td, 1 H), 6.64 (d, 1H), 6.56 (d, 1H), 6.40 (d, 1H), 6.10 (d, 1H), 6.03 (dd, 1H), 4.63 (m, 1H), 4.22 (d, 1H), 4.16 (m, 1H), 4.03 (d, 1H), 3.78 (s, 3H), 3.48 (td, 1H), 3.29 (dt, 1H), 2.89 (m, 4H), 2.71 (ddd, 1H), 2.42 (b, 1H, OH), 2.13 (td, 1H), 2.01 (ddd, 1H), 1.51 (ddd, 1H);
¹³C-NMR (CDCl₃) : δ 145.8 (s), 144.2 (s), 142.8 (s), 137.9 (s), 133.2 (s), 139.5 (s), 130.5 (d), 130.3 (s), 128.8 (d), 128.5 (s), 127.7 (d), 126.8 (d), 126.7 (d), 124.1 (d), 122.3 (d), 120.9 (d), 116.3 (d); 110. 9 (d), 88.7 (d), 62.0 (t), 57.3 (t), 55.7 (q), 52.5 (t), 52.0 (t), 48.4 (s), 32.5 (t), 30.7 (t), 29.9 (t);

### Beispiel 41

### (4aS,6R,8aS)-4a,5,9,10,11,12-Hexahydro-3-methoxy-11-(4-bromo-benzoyl)-6H-[1]benzofuro[3a,3,2-ef][2]benzazepin-6-ol (Ia Y_{L}=OH, Y₂=H, X=H, Z₁=C₁₁H₁₃O)

2,0 g (-)-Norgalanthamin , welches gemäß der WO-A-01/74820 hergestellt wurde, sowie 4,0 g Kaliumcarbonat und 1,65 g 4-Brombenzoesäurechlorid werden in 70 ml Acetonitril bei Siede_ temperatur gerührt. Nach 3 Stunden wird das Lösungsmittel abdestilliert, der Rückstand mit 100 ml Wasser und 100 ml Ethylacetat versetzt. Nach dem Abtrennen der organischen Phase wird die wässrige Phase mit 2x50 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=95:5) gereinigt
Ausbeute: 3,3 g (99 % d. Th)
Smp: 98-112°C
R_{f}: 0,35 (Chloroform:MeOH=9:1)
¹H-NMR (CDCl₃) δ 7.51 (d, 2H), 7.13 (d, 2H), 6.65 (d, 1H), 6.24 (d, 1H), 6.08 (td, 1H), 5.98 (d, 1H), 4.90 (b, 1H), 4.68 (b, 1H), 4.42 (s, 1H), 4.18 (d, 1H), 3.85 (s, 3H), 3.45 (m, 1H), 2.73 (dt, 1H), 2.10 (m, 2H), 1.93 (d, 1H), 1.71 (b, 1H);
¹³C-NMR (CDCl₃): δ 170.9 (s), 147.2 (s), 145.0 (s), 135.5 (s), 133.1 (s), 131.8 (2d), 128.9 (2d), 182.5 (d), 128.4 (s), 126.8 (d), 124.3 (s), 121.0 (d), 112.0 (d), 88.8 (d), 62.2 (t), 56.3 (q), 54.9 (t), 48.7 (s), 44.5 (t), 36.6 (t), 30.2 (t);

### Beispiel 42

### (4aS,6R,8aS)-11-(4,6-dichloro-1,3,5,-triazin-2-yl)-3-methoxy-5,6,9,10,11,12-hexahydro-4aH-[1]benzofuro[3a,3,2-ef][2]benzazepin-6-ol (Ia Y₁=OH, Y₂=H, X=H, Z₁=C₁₁H₁₃O)

Eine Lösung von 1,32 g Cyanurchlorid in 32 ml Aceton wird auf 70 ml Eiswasser gegossen und bei 0 °C mit 2,0 g (-)-Norgalanthamin, welches gemäß der WO-A-01/74820 hergestellt wurde, versetzt. Die Reaktionsmischung wird mit 4,0 ml 2N Natriumhydroxid-Lösung versetzt und bei Siede-temperatur gerührt. Nach 40 Stunden wird das Reaktionsgemisch auf Raumtemperatur gekühlt und mit 60 ml Ethylacetat versetzt. Nach dem Abtrennen der organischen Phase wird die wässrige Mutterlauge mit 2x60 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=98:2) gereinigt
Ausbeute: 1,58 g (51 % d. Th)
Smp: 245-149 °C
R_{f}: 0,75 (Chloroform:MeOH=9:1)
¹H-NMR (DMSO): δ 6.79 (b, 2H), 6.19 (d, 1H), 5.81 (dd, 1H), 5.12 (d, 1H), 4.79 (d, 1H), 4.58 (d, 1H), 4.49 (b, 1H), 4.10 (b, 1H), 3.79 (m, 1H), 3.72 (s, 3H), 2.29 (d, 1H), 2.09 (m, 1H), 1.80 (m, 2H);
¹³C-NMR (DMSO): δ 170.0 (s), 169.9 (s), 164.3 (s), 147.9 (s), 144.7 (s) 132.9 (s), 129.4 (d), 127.6 (d), 127.5 (s), 121.8 (d), 112.2 (d), 87.0 (d), 60.4 (d), 56.4 (q), 52.1 (t), 48.5 (s), 46.6 (t), 40.0 (t), 31.4 (t);

### Beispiel 43

### (4aS,6R,8aS)-11-(4-Brombenzyl)-4a,5,9,10,11,12-Hexahydro-3-methoxy-6H-[1]benzofuro[3a,3,2-ef][2]benzazepin-6-ol (Ia Y₁=OH, Y₂=H, X=H, Z₁=C₇H₆Br)

3,0 g (-)-Norgalanthamin, welches gemäß der WO-A-01/74820 hergestellt wurde, sowie 3,0 g Kaliumcarbonat und 2,5 g 4-Brombenzylbromid werden in 70 ml Acetonitril bei Raumtemperatur gerührt. Nach 24 Stunden wird das Lösungsmittel unter Vakuum entfernt und der Rückstand mit 100 ml Wasser und 40 ml Ethylacetat versetzt. Nach dem Abtrennen der organischen Phase wird die wässrige Mutterlauge mit 2x40 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=99:1) gereinigt
Ausbeute: 3,0 g (70,2 % d. Th)
Smp: 148-149 °C
R_{f}: 0,8 (Chloroform:MeOH=9:1)
¹H-NMR (CDCl₃) : δ 7.53 (d, 2H), 7.18 (d, 2H), 6.65 (d, 1H), 6.40 (d, 1H), 6.15 (d, 1H), 6.03 (d, 1H), 4.68 (b, 1H), 4.12 (m, 1H), 3.85 (s, 3H), 3.66 (d, 1H), 3.61 (s, 2H), 3.41 (t, 1H), 3.18 (d, 1H), 2.71 (dd, 1H), 2.44 (d, 1H), 2.15 (dd, 1H), 2.03 (dd, 1H), 1.65 (d, 1H);
¹³C-NMR (CDCl₃): δ 146.3 (s), 144.5 (s), 138.4 (s), 133.7 (s), 131.8 (2d), 131.0 (2d), 129.8 (s), 128.1 (d), 127.2 (d), 122.5 (d), 121.2 (s), 111.6 (d), 89.2 (d), 62.5 (t), 57.7(t), 56.3 (q), 56.0 (t), 52.2 (t), 48.9 (s), 33.9 (t), 30.4 (t);

### Beispiel 44

### Ethyl-2-((4aS,6R,8aS)-6-hydroxy-3-methoxy-5,6,9,10-tetrahydro-4aH-[1]benzofuro[3a,3,2-ef][2]benzazepin-11(12H)-yl)acetate (Ia Y₁=OH, Y₂=H, X=H, Z₁=C₄H₇O₂)

2,0 g (-)-Norgalanthamin, welches gemäß der WO-A-01/74820 hergestellt wurde, sowie 4,0 g Kaliumcarbonat und 1,0 ml Bromessigsäureethylester werden in 50 ml Tetrahydrofuran bei Raumtemperatur gerührt. Nach 16 Stunden wird der Niederschlag abfiltriert, das Filtrat mit 100 ml Wasser und 50 ml Ethylacetat versetzt. Nach dem Abtrennen der organischen Phase wird die wässrige Mutterlauge mit 2x50 ml Ethylacetat extrahiert. Die vereinigten organischen Phasen werden über Natriumsulfat getrocknet, filtriert und unter Vakuum eingeengt. Das Produkt wird säulenchromatographisch (reines Ethylacetat) gereinigt.
Ausbeute: 1,46 g (55,5 % d. Th)
Smp: 75-78 °C
R_{f}: 0,8 (Ethylacetat)
¹H-NMR (DMSO): δ 6.69 (d, 1H), 6.49 (d, 1H), 6.08 (d, 1H), 5.80 (dd, 1H), 4.49 (b, 1H), 4.21 (m, 2H), 4.08 (m, 2H), 3.75 (s, 3H), 3.68 (m, 1H), 3.32 (m, 2H), 2.23 (d, 1H), 3.00 (d, 1H), 2.28 (d, 1H), 2.07 (td, 1H), 1.93 (t, 1H), 1.58 (d, 1H), 1.18 (t, 3H);
¹³C-NMR (DMSO): δ 171.4 (s), 147.1 (s), 144.2 (s), 133.6 (s), 130.1 (s), 129.2 (d), 127.8 (d), 121.7 (d), 112.2 (d), 87.7 (d), 60.7 (d), 60.6 (t), 58.0 (t), 56.3 (q), 54.6 (t), 52.5 (t), 48.6 (s), 35.1 (t), 31.9 (t), 15.0 (q);

### Beispiel 45

### 2-((4aS,6R,8aS)-6-Hydroxy-3-methoxy-5,6,9,10-tetrahydro-4aH-[1]benzofuro[3a,3,2-ef][2]benzazepin-11(12H)-yl)acetic acid (Ia Y₁=OH, Y₂=H, X=H, Z₁=C₂H₃O₂)

### Stufe 1

2,0 g (-)-Norgalanthamin, welches gemäß der WO-A-01/74820 hergestellt wurde, sowie 4,0 g Kaliumcarbonat und 1,0 ml Bromessigsäureethylester werden in 50 ml Tetrahydrofuran bei Raumtemperatur gerührt. Nach 16 Stunden wird der Niederschlag abgetrennt, das Lösungsmittel unter Vakuum entfernt und der Rückstand mit 100 ml Wasser und 50 ml Ethylacetat versetzt. Nach dem Abtrennen der organischen Phase wird die wässrige Mutterlauge mit 2x50 ml Ethylacetat extrahiert. Die vereinigten organischen Phasen werden über Natriumsulfat getrocknet, filtriert und unter Vakuum eingeengt. Man erhält 2,8 g schaumartiges Material.

### Stufe2

1,6 g des aus Stufe 1 erhaltenen Produktes werden in 24 ml Ethanol aufgelöst, mit 3,2 ml 2N Natriumhydroxid-Lösung versetzt und bei Raumtemperatur gerührt. Nach 30 min wird die klare Lösung mit 4,8 g IRA-120 Ionenaustauscher versetzt und noch 10 min weitergerührt. Der Ionenaustauscher wird abfiltriert, das Filtrat im Vakuum eingeengt und der Rückstand aus einer Mischung von Methanol und tert.-Butyl-methylether (MTBE) auskristallisiert.
Ausbeute: 0,8 g weißes Pulver
Smp: 144-161°C
R_{f}: 0,2 (Chloroform:MeOH=6:4)
¹H-NMR (DMSO): δ 6.69 (d, 1H), 6.49 (d, 1H), 6.08 (d, 1H), 5.80 (dd, 1H), 4.49 (b, 1H), 4.21 (m, 2H), 4.08 (m, 2H), 3.75 (s, 3H), 3.68 (m, 1H), 3.32 (m, 2H), 2.23 (d, 1H), 3.00 (d, 1H), 2.28 (d, 1H), 2.07 (td, 1H), 1.93 (t, 1H), 1.58 (d, 1H), 1.18 (t, 3H);
¹³C-NMR (DMSO): δ 171.4 (s), 147.1 (s), 144.2 (s), 133.6 (s), 130.1 (s), 129.2 (d), 127.8 (d), 121.7 (d), 112.2 (d), 87.7 (d), 60.7 (d), 60.6 (t), 58.0 (t), 56.3 (q), 54.6 (t), 52.5 (t), 48.6 (s), 35.1 (t), 31.9 (t), 15.0 (q);

### Beispiel 46

### (4aS,6R,8aS)-4a,5,9,10,11,12-Hexahydro-3-methoxy-11-(2-methyl-prop-2-enyl)-6H-[1]benzofuro[3a,3,2-ef] [2]benzazepin-6-ol, Hydrochloride (Ia Y₁=OH, Y₂=H, X=H, Z₁=C₂H₃O₂)

2,0 g (-)-Norgalanthamin, welches gemäß der WO-A-01/74820 hergestellt wurde, sowie 2,02 g Kaliumcarbonat, 1,27 g Kaliumjodid und 0,85 ml 3-Chlor-2-methyl-1-propen werden in 80 ml Aceton bei Rückflusstemperatur gerührt. Nach 4 Stunden wird die Reaktionsmischung auf Raumtemperatur abgekühlt, das Lösungsmittel im Vakuum abdestilliert und der Rückstand mit 200 ml 2N HCl und 50 ml Ethylacetat versetzt. Nach dem Abtrennen wird die organische Phase entsorgt. Die wässrige Phase wird mit 30%-iger Natriumhydroxid-Lösung basisch eingestellt und mit 3x100 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=95:5) gereinigt. Das erhaltene Öl wird in Ethanol aufgelöst und das Hydrochlorid-Salz mit etherischer HCl ausgefällt.
Ausbeute: 2,38 g (99 % d. Th)
Smp: 233-234 °C
R_{f}: 0,8 (Chloroform:MeOH:Ammoniak-Lösung=89:10:1)
¹H-NMR (DMSO): δ 6.88 (d, 1H), 6.70 (d, 1H), 6.17 (d, 1H), 5.93 (d, 1H), 5.39 (d, 1H), 5.20 (d, 1H), 4.62 (m, 2H), 4.28 (m, 1H), 4.12 (b, 1H), 3.95 (b, 1H), 3.81 (s, 3H), 3.62 (m, 3H), 2.29 (d, 1H), 2.10 (d, 2H), 1.93 (d, 3H), 1.58 (d, 1H);
¹³C-NMR (DMSO): δ 147.4 (s), 145.8 (s), 136.5 (s), 134.1 (s), 130.9 (d), 126.3 (d), 123.5 (d), 122.6 (s), 112.8 (d), 87.4 (d), 67.9 (t), 64.2 (t), 57.7 (d), 56.6 (t), 56.4 (q), 50.5 (t), 47.6 (s), 31.9 (t), 26.0 (t), 22.2 (q);

### Beispiel 47

### Ethyl-3-((4aS,6R,8aS)-6-hydroxy-3-methoxy-5,6,9,10-tetrahydro-4aH-[1]benzofuro[3a,3,2-ef][2]benzazepin-11(12H)-yl)propanoate, Hydrochloride (Ia Y₁=OH, Y₂=H, X=H, Z₁=C₅H₉O₂)

0,55 g (-)-Norgalanthamin, welches gemäß der WO-A-01/74820 hergestellt wurde, sowie 0,3 ml Acrylsäureethylester werden in 20 ml abs. Ethanol bei Rückflusstemperatur gerührt. Nach 72 Stunden wird das Lösungsmittel abdestilliert und das Produkt säulenchromatographisch (Chloroform:MeOH=95:5) gereinigt. Das erhaltene Öl wird in Chloroform aufgelöst und das Hydrochlorid-Salz mit etherischer HCl ausgefällt.
Ausbeute: 0,5 g (60,6 % d. Th)
R_{f}: 0,6 (Chloroform:MeOH=95:5)
¹H-NMR (DMSO): δ 6.88 (d, 1H), 6.70 (d, 1H), 6.17 (d, 1H), 5.93 (d, 1H), 5.39 (d, 1H), 5.20 (d, 1H), 4.62 (m, 2H), 4.28 (m, 1H), 4.12 (b, 1H), 3.95 (b, 1H), 3.81 (s, 3H), 3.62 (m, 3H), 2.29 (d, 1H), 2.10 (d, 2H), 1.93 (d, 3H), 1.58 (d, 1H);
¹³C-NMR (DMSO): δ 147.4 (s), 145.8 (s), 136.5 (s), 134.1 (s), 130.9 (d), 126.3 (d), 123.5 (d), 122.6 (s), 112.8 (d), 87.4 (d), 67.9 (t), 64.2 (t), 57.7 (d), 56.6 (t), 56.4 (q), 50.5 (t), 47.6 (s), 31.9 (t), 26.0 (t), 22.2 (q);

### Beispiel 48

### 1-[(4aS,6R,8aS)-6-Hydroxy-3-methoxy-5,6,9,10-tetrahydro-4aH-[1]benzofuro[3a,3,2-ef][2]benzazepin-11(12H)-yl]-2-(4-morpholinyl)ethan-1-on (Ia Y₁=OH, Y₂=H, X=H, Z₁=C₈H₁₄N₃O)

3,0 g (-)-Norgalanthamin, welches gemäß der WO-A-01/74820 hergestellt wurde, sowie 1,86 ml Triethylamin und 0,9 ml Chroracetylchlorid werden in 150 ml Tetrahydrofuran bei 0 °C gerührt. Nach 10 min wird die Reaktionsmischung mit 3,0 g Kaliumcarbonat und 1,2 ml Morpholin versetzt und bei 90°C weitergerührt. Nach 60 Stunden wird das Lösungsmittel abdestilliert, der Rückstand mit 50 ml Wasser und 50 ml Chloroform versetzt. Nach dem Abtrennen der organischen Phase wird die wässrige Phase mit 2x30 ml Chloroform extrahiert. Die vereinigten organischen Phasen werden über Natriumsulfat getrocknet, filtriert und unter Vakuum eingeengt. Das Produkt wird säulenchromatographisch (Chloroform:MeOH=98:2) gereinigt.
Ausbeute: 2,5 g (56,8 % d. Th)
Smp: 92-101 °C
R_{f}: 0,45 (Chloroform:MeOH=9:1)
¹H-NMR (CDCl₃): δ 6.67 (m, 2H), 6.01 (m, 2H), 4.99 (d, 1H), 4.63 (d, 1H), 4.55 (b, 1H), 4.37 (d, 1H), 4.12 (b, 1H), 3.82 (s, 3H), 3.67 (m, 4H), 3.25 (d, 1H), 3.14 (m, 2H), 3.00 (d, 1H), 2.48 (m, 4H), 2.01 (dd, 1H), 1.88 (t, 1H), 1.75 (d, 1H);
¹³C-NMR (CDCl₃): δ 169.2 (s), 147.3 (s), 145.0 (s), 132.9 (s), 128.9 (s), 128.8 (d), 126.8 (d), 122.4 (d), 111.7 (d), 88.7 (d), 67.1 (2t), 62.1 (d), 56.3 (q), 54.1 (2t), 52.7 (t), 48.7 (s), 45.5 (t), 38.9 (t), 36.1 (t), 30.3 (t);

### Beispiel 49

### 1-[(4aS,6R,8aS)-6-Hydroxy-3-methoxy-5,6,9,10-tetrahydro-4aH-[1]benzofuro[3a,3,2-ef][2]benzazepin-11(12H)-yl]-2-(diethylamino)ethan-1-on (Ia Y₁=OH, Y₂=H, X=H, Z₁=C₆H₁₂NO)

3,0 g (-)-Norgalanthamin, welches gemäß der WO-A-01/74820 hergestellt wurde, sowie 1,86 ml Triethylamin und 0,9 ml Chroracetylchlorid werden in 150 ml Tetrahydrofuran bei 0 °C gerührt. Nach 10 min wird die Reaktionsmischung mit 3,0 g Kaliumcarbonat und 0,75 ml Diethylamin versetzt und bei 90°C weitergerührt. Nach 24 Stunden wird das Lösungsmittel abdestilliert, der Rückstand mit 50 ml Wasser und 50 ml Chloroform versetzt. Nach dem Abtrennen der organischen Phase wird die wässrige Phase mit 2x30 ml Chloroform extrahiert. Die vereinigten organischen Phasen werden über Natriumsulfat getrocknet, filtriert und im Vakuum eingeengt. Das Produkt wird säulenchromatographisch (Chloroform:MeOH=95:5) gereinigt.
Ausbeute: 2,0 g (48,5 % d. Th)
Smp: 114-126°C
R_{f}: 0,45- (Chloroform:MeOH=9:1)
¹H-NMR (CDCl₃): δ 6.68 (m, 2H), 6.01 (m, 2H), 5.19 (d, 1H), 4.60 (m, 1H), 4.33 (d, 1H), 4.09 (b, 1H), 3.78 (s, 3H), 3.37 (d, 1H), 3.21 (m, 1H), 2.95 (d, 1H), 2.64 (m, 3H), 2.49 (m, 3H), 2.03 (dd, 1H), 1.92 (t, 1H), 1.75 (d, 1H), 1.01 (m, 6H);
¹³C-NMR (CDCl₃): δ 170.8 (s), 147.2 (s), 145.0 (s), 132.9 (s), 129.1 (s), 128.6 (d), 126.9 (d), 121.0 (d), 111.7 (d), 88.7 (d), 62.3 (d), 56.3 (t), 56.2 (q), 52.4 (t), 48.7 (2t), 47.8 (s), 38.9 (t), 36.1 (t), 30.3 (t), 12.0 (2q);

### Beispiel 50

### (4aS,6R,8aS)-4a,5,9,10,11,12-Hexahydro-3-methoxy-11-[3-(1-piperidinyl)butyl]-6H-benzofuro[3a,3,2-ef][2]benzazepin-6-ol, (+) Di-O-p-toluoyl tartrate, (Ia Y₁=OH, Y₂=H, X=H, Z₁=C₉H₁₈N)

### Stufe 1:

4,1 g 4-Brombutansäurepiperidinamid (17,5 mmol) werden in 100 ml Acetonitril gelöst. Zu dieser Lösung werden 3,8 g (-) Norgalanthamin Hydrochlorid, welches gemäß der WO-A-01/74820 hergestellt wurde, sowie 9,7g Kaliumcarbonat zugegeben und 30 Stunden bei 80°C gerührt. Nach dem Abfiltrieren des Kaliumcarbonats wird das Lösungsmittel abdestilliert und der Rückstand in 100 ml Toluol und 100 ml In HCl aufgenommen. Die wässrige Phase wird mit 30%iger Natriumhydroxid-Lösung basisch eingestellt und 3x mit je 40 ml Ethylacetat ausgeschüttelt. Die vereinigten organischen Phasen werden über Natriumsulfat getrocknet, filtriert und eingedampft. Die erhaltenen 4,4 g an braunem Öl werden an 200 g Kieselgel mit dem Laufmittel Chloroform: Methanol= 98:2 säulenchromatographisch gereinigt.
Ausbeute: 1,6 g (30 % d. Th.)

### Stufe 2:

3,6 g Stufe 1 werden in 50ml Tetrahydrofuran gelöst, auf 0°C gekühlt und 705 mg Lithiumaluminiumhydrid in Portionen während 20 Minuten zugegeben. Nach beendeter Zugabe wird auf Raumtemperatur erwärmt und 1,5 Stunden gerührt. Die Reaktion wird tropfenweise mit Wasser gequencht, der entstandene Niederschlag abfiltriert und mit 10ml Tetrahydrofuran gewaschen. Nach dem Trocknen der Lösung mit Natriumsulfat wird diese filtriert und das Lösungsmittel abgezogen. Die erhaltenen 3,4 g werden an 200 g Kieselgel mit dem Laufmittel Chloroform:Methanol:Ammoniak-Lösung=90:9:1 gesäult. Die erhaltenen 2,4 g werden in 80 ml Ethylacetat gelöst und mit 2,5 g (+)-Di-p-toluyl-D-Weinsäure in 30ml Ethylacetat gefällt, filtriert und mit 10 ml Ethylacetat gewaschen.
Ausbeute: 4,4 g (78,7% d. Th.) farblose Kristalle
¹H-NMR (CDCl₃): δ 6.67 (d, 1H), 6.61 (d, 1H), 6.12 (d, 1H), 6.01 (dd, 1H), 4.63 (m, 1H), 4.15 (m, 2H), 3.88 (s, 3H), 3.82 (d, 1H), 3.35 (d, 1H), 3.18 (d, 1H), 2.70 (dd, 1H), 2.49 (m, 4H), 2.31 (m, 4H), 2.02 (m, 4H), 1.71 (m, 4H), 1.49 (m, 5H);
¹³C-NMR (CDCl₃): δ 146.1 (s), 144.4 (s), 133.5 (s), 129.9 (s), 127.9 (d), 127.4 (d), 122.4 (d), 111.5 (d), 89.1 (d), 66.1 (t), 62.5 (d), 59.7 (t), 58.3 (t), 56.2 (q), 54.9 (2t), 51.9 (t), 48.8 (s), 33.4 (t), 30.3 (t), 26.3 (2t), 26.0 (t), 25.1 (t), 24.9 (t);

### Beispiel 51

### 3-((4aS,6R,8aS)-1-bromo-6-hydroxy-3-methoxy-5,6,9,10-tetrahydro-4aH-[1]benzofuro[3a,3,2-ef][2]benzazepin-11(12H)-yl)propanenitrile (Ia Y₁=OH, Y₂=H, X=H, Z₁=C₃H₄N)

2,0 g (-)-Norgalanthamin, welches gemäß der WO-A-01/74820 hergestellt wurde, sowie 2,0 g CaCl₂ und 0,5 ml Acrylnitril werden in 200 ml Ethanol bei Siedetemperatur gerührt. Nach 8 Stunden wird das Lösungsmittel abdestilliert, der Rückstand in 500 ml 2 N HCl aufgenommen und mit 3x200 ml Ethylacetat extrahiert. Die wässrige Mutterlauge wird mit 25%-iger Ammoniak-Lösung basisch eingestellt und mit 3x 200 ml Ethylenchlorid extrahiert. Die vereinigten organischen Phasen werden über Natriumsulfat getrocknet, filtriert und im Vakuum eingeengt. Das Produkt wird säulenchromatographisch (Chloroform:MeOH=9:1) gereinigt.
Ausbeute: 1,7 g (62 % d. Th)
Smp: 69-72 °C
R_{f}: 0,45 (Chloroform:MeOH:Ammoniak-Lösung=90:9:1)
¹H-NMR (CDCl₃): δ 6.67 (d, 1H), 6.60 (d, 1H), 6.06 (d, 1H), 5.98 (dd, 1H), 4.69 (b, 1H), 4.21 (d, 1H), 4.10 (m, 1H), 3.82 (s, 3H), 3.79 (d, 1H), 3.45 (t, 1H), 3.27 (d, 1H), 2.80 (m, 2H), 2.67 (dd, 1H), 2.43 (m, 2H), 1.99 (m, 2H), 1.59 (d, 1H);
¹³C-NMR (CDCl₃) : δ 146.5 (s), 144.8 (s), 133.4 (s), 129.0 (s), 128.3 (d), 126.9 (d), 122.4 (d), 119.3 (s), 111.7 (d), 89.0 (d), 62.3 (d), 57.4 (t), 56.3 (q), 52.1 (t), 48.9 (s), 47.0 (t), 33.5 (t), 30.4 (t),17.2 (t);

### Beispiel 52

### (4aS,6R,8aS)-11-((3-dimethylamino)propyl)-3-methoxy-5,6,9,10,11,12-hexahydro-4aH-[1] benzofuro[3a,3,2-ef][2]benzazepin-6-ol (Ia Y₁=OH, Y₂=H, X=H, Z₁=C₅H₁₂N)

3,0 g (-)-Norgalanthamin HCl, welches gemäß der WO-A-01/74820 hergestellt wurde, sowie 5,0 g Kaliumcarbonat und 2,1 g 3-Dimethylaminopropylchlorid HCl werden in 70 ml Acetonitril bei Siedetemperatur gerührt. Nach 28 Stunden wird der Niederschlag abfiltriert und das Lösungsmittel im Vakuum entfernt. Das Produkt wird säulenchromatographisch (Chloroform:MeOH=9:1) gereinigt.
Ausbeute: 2,35 g g (59,8 % d. Th) braunes Öl.
R_{f}: 0,35 (Chloroform:MeOH=9:1)
¹H-NMR (DMSO): δ 6.67 (d, 1H), 6.60 (d, 1H), 6.11 (d, 1H), 5.94 (dd, 1H), 4.59 (b, 1H), 4.13 (m, 2H), 3.82 (s, 3H), 3.78 (d, 1H), 3.21 (m, 11H), 2.52 (m, 2H), 2.29 (m, 1H), 2.05 (d, 1H), 2.65 (m, 2H), 1.51 (d, 1H);
¹³C-NMR (DMSO): δ 146.3 (s), 144.2 (s), 133.5 (s), 129.8 (s), 128.0 (d), 127.5 (d), 122.2 (d), 111.6 (d), 88.7 (d), 61.9 (d), 58.0 (t), 57.8 (t), 56.2 (q), 52.0 (t), 50.0 (t), 48.7 (s), 45.7 (2q), 33.4 (t), 30.7 (t), 25.8 (t);

### Beispiel 53

### (4aS,6R,8aS)-N11-cyclohexyl-6-hydroxy-3-methoxy-5,6,9,10-tetrahydro-4aH-[1]benzofuro[3a,3,2-ef][2]benzazepin-11(12H)-carbonoc acid isopropylamide (Ia Y₁=OH, Y₂=H, X=H, Z₁=C₇H₁₂NO)

2,0 g (-)-Norgalanthamin, welches gemäß der WO-A-01/74820 hergestellt wurde, sowie 0,92 g Cyclohexylisocyanat werden in 100 ml Toluol aufgelöst und bei Siedetemperatur gerührt. Nach 5 Stunden wird das Lösungsmittel im Vakuum abdestilliert, und der Rückstand mit 200 ml 2 N HCl und 100 ml Diethylether versetzt. Nach dem Abtrennen der organischen Phase wird die wässrige Mutterlauge mit 25 %-iger Ammoniak-Lösung basisch eingestellt und mit 3x100 ml Ethylacetat extrahiert. Die vereinigten organischen Phasen werden über Natriumsulfat getrocknet, filtriert und das Lösungsmittel wird unter Vakuum abdestilliert. Der Rückstand wird aus Ethanol kristallisiert.
Ausbeute: 1,97 g (67,5 % d. Th)
Smp: 168-170
R_{f}: 0,6 (Chloroform:MeOH=9:1)
¹H-NMR (CDCl₃) : δ 6.69 (d, 1H), 6.62 (d, 1H), 5.95 (m, 2H), 4.57 (b, 1H), 4.46 (d, 1H), 4.31 (d, 2H), 4.12 (b, 1H), 3.80 (s, 3H), 3.41 (m, 1H), 3.32 (t, 1H), 2.63 (d, 1H), 2.03 (dd, 1H), 1.91 (d, 2H), 1.70 (d, 2H), 1.55 (m, 2H), 1.25 (m, 2H), 1.09 (m, 2H), 0.95 (m, 2H);
¹³C-NMR (CDCl₃) : δ 156.9 (s), 147.3 (s), 145.0 (s), 132.9 (s), 129.6 (s), 128.4 (d), 126.9 (d), 120.7 (d), 111.5 (d), 88.8 (d), 62.2 (d), 56.3 (q), 52.0 (t), 49.6 (d), 48.9 (t), 46.0 (s), 36.9 (t), 34.2 (t), 33.9 (t), 30.2 (t),26.0 (t), 25.2 (t), 25.1 (t);

### Beispiel 54

### 1-[(4aS,6R,8aS)-6-hydroxy-3-methoxy-5,6,9,10-tetrahydro-4aH-[1]benzofuro[3a,3,2-ef][2]benzazepin-11(12H)-yl]-2-chlorethan-1-on (Ia Y₁=OH, Y₂=H, X=H, Z₁=C₂H₂ClO)

Eine Lösung von 3,0 g (-)-Norgalanthamin, welches gemäß der WO-A-01/74820 hergestellt wurde, sowie 1,9 ml Triethylamin in 150 ml Tetrahydrofuran wird bei 0 °C mit 0,93 ml Chloracetylchrorid versetzt. Nach 10 min wird das Lösungsmittel im Vakuum entfernt, der Rückstand mit 100 ml Wasser, 10 ml 2 N Salzsäure versetzt und mit 3x 30 ml Diethylether exrahiert Die vereinigten organischen Phasen werden über Natriumsulfat getrocknet, filtriert und das Lösungsmittel wird unter Vakuum abdestilliert.
Ausbeute: 1,42 g (37,1 % d. Th)
Smp: 88-90 °C
R_{f}: 0,8 (Chloroform:MeOH=9:1)
¹H-NMR (CDCl₃): δ 6.73 (m, 2H), 6.02 (m, 2H), 4.69 (d, 1H), 4.65 (d, 1H), 4.52 (d, 1H), 4.19 (d, 1H), 4.09 (m, 2H), 3.95 (d, 1H), 3.85 (s, 3H), 3.30 (t, 1H), 2.73 (d, 1H), 2.09 (dd, 1H), 1.91 (d, 1H), 1.71 (d, 1H);
¹³C-NMR (CDCl₃): 5 166 .5 (s), 147.5 (s), 145.5 (s), 132.8 (s), 129.0 (s), 128.2 (d), 126.5 (d), 122.6 (d), 111.7 (d), 88.8 (d), 62.2 (d), 56.4 (q), 53.4 (t), 48.7 (d), 46.0 (s), 41.9 (t), 35.9 (t), 30.2 (t);

### Beispiel 55

### (4aR,6S,8aR)-6-Hydroxy-3-methoxy-11-methyl-4a,5,9,10-tetrahydro-6H-benzofuro[3a,3,2-ef][2]benzazepinium Bromide

Eine Lösung von 2,0 g (+)-Galanthamin, hergestell nach Kametani, Heterocycles 4, 1111, 1976, in 20 ml Chloroform wird intensiv gerührt und mit einer Lösung von 1,26 g N-Bromsuccinimid in 20 ml Chloroform tropfenweise bei Raumtemperatur versetzt. Nach einer Stunde wird der gebildete Niederschlag abgetrennt, mit Chloroform gewaschen und im Vakuumtrockenschrank bei 50 °C getrocknet. Das Produkt wird aus Ethanol umkristallisiert.
Ausbeute: 2,28 g (90,2 % d. Th)
Smp: 223-229 °C
R_{f}: 0,2 (Chloroform:MeOH:Ammoniak-Lösung=90:9:1)
¹H-NMR (DMSO): δ 9.15 (s, 1H), 7.57 (d, 1H), 7.20 (d, 1H), 5.89 (dd, 1H), 5.72 (d,1H), 4.67 (b, 1H), 4.61 (d, 1H), 4.13 (m, 3H), 3.95 (s, 3H), 3.80 (s, 3H), 2.35 (d, 1H), 2.15 (m, 2H);
¹³C-NMR (DMSO): δ 167.3 (d), 151.3 (s), 146.2 (s), 136.9 (s), 133.0 (d), 129.8 (d), 126.4 (d), 115.0 (s), 112.9 (d), 86.9 (d), 58.9 (d), 56.4 (q), 54.0 (t), 51.5 (t), 45.9 (q), 40.7 (t), 31.1 (t), 29.7 (t);

### Beispiel 56

### (4aR, 6R, 8aR)-6-Hydroxy-3-methoxy-11-methyl-4a,5,9,10-tetrahydro-6H-benzofuro[3a, 3, 2-ef][2]benzazepinium Bromide

Zu einer intensiv gerührten Lösung von 2,0 g (+)-EpiGalanthamin, hergestellt nach J. Chem. Soc. 806, 1962, in 80 ml Chloroform werden 1,35 g N-Bromsuccinimid bei Raumtemperatur zudosiert. Nach einer Stunde wird der gebildete Niederschlag abgetrennt, mit Chloroform gewaschen und im Vakuumtrockenschrank bei 50°C getrocknet. Das Produkt wird aus Ethanol umkristallisiert.
Ausbeute: 2,09 g (82,7 % d. Th)
Smp: 236-244 °C
R_{f}: 0,2 (Chloroform:MeOH:Ammoniak-Lösung=90:9:1)
¹H-NMR (DMSO): δ 9,15 (s, 1H), 7.58 (d, 1H), 7.20 (d, 1H), 5.85 (dd, 1H), 5.74 (d, 1H), 5.15 (d, 1H), 4.79 (b, 1H), 4.30 (m, 1H), 4.13 (m, 2H), 3.93 (s, 3H), 3.80 (s, 3H), 2.55 (d, 1H), 2.20 (m, 1H), 1.73 (dt, 1H);
¹³C-NMR (DMSO): δ 168.1 (d), 152.1 (s), 147.3 (s), 138.1 (s), 135.4 (d), 133.9 (d), 126.9 (d), 116.0 (s), 113.9 (d), 88.9 (d), 61.7 (d), 57.3 (q), 55.1 (t), 52.3 (t), 47.1 (q), 41.0 (t), 32.3 (t), 31.7 (t);

### Beispiel 57

### 4aS,6R,8aS)-1-Bromo-4a,5,9,10,11,12-hexahydro-11-(2-(morpholin-4-yl)-ethyl)-3-methoxy-6H-[1]benzofuro[3a,3,2-ef][2]benzazepin-6-ol (Ia, Y₁=OH, Y₂=H, X=Br, Z₁= C₆H₁₂NO)

2,0 g (-)-BromNorgalanthamin (Ia Y₁=OH, Y₂=H, X=Br, Z₁=H), 2,35 g Kaliumcarbonat und 1,11 g N-(2-Chlorethyl)-morpholin Hydrochlorid werden in 30 ml Acetonitril bei Rückflusstemperatur gerührt. Nach 48 Stunden wird die Reaktionsmischung auf Raumtemperatur abgekühlt, das Lösungsmittel im Vakuum abdestilliert und der Rückstand mit 200 ml 2N HCl und 40 ml Ethylacetat versetzt. Nach dem Abtrennen wird die organische Phase entsorgt. Die wässrige Phase wird mit Ammoniak-Lösung basisch eingestellt und mit 3x40 ml Ethylacetat extrahiert. Die vereinigten organischen Phasen werden über Natriumsulfat getrocknet, filtriert und im Vakuum eingeengt. Das Produkt wird säulenchromatographisch (Chloroform:MeOH=95:5) gereinigt.
Ausbeute: 1,39 g (53 % d. Th), weißer Schaum
R_{f}: 0,2 (Chloroform:MeOH:Ammoniak-Lösung=90:9:1)
¹H-NMR (DMSO): δ 6.91 (s, 1H), 6.15 (d, 1H), 6.03 (dd, 1H), 4.51 (b, 1H), 4.41 (d, 1H), 4.16 (b, 1H), 4.05 (d, 1H), 3.85 (s, 3H), 3.71 (t, 4H), 3.39 (t, 1H), 3.15 (d, 1H), 2.69 (m, 3H), 2.51 (m, 5H), 2.03 (m, 3H), 1.55 (d, 1H);
¹³C-NMR (CDCl₃): δ 145.9 (s), 144.7 (s), 134.6 (s), 128.5 (d), 128.4 (s), 127.4 (d), 116.3 (d), 114.9 (s), 89.2 (d), 67.3 (2t), 62.3 (d), 57.3 (2t), 56.6 (q), 56.6 (t), 54.6 (2t), 52.6 (t), 49.4 (t), 33.6 (t), 30.2 (t);

### Beispiel 58

### (4aR,6R,8aRS)-1-Bromo-4a,5,9,10,11,12-hexahydro-11-(2-(morpholin-4-yl)-ethyl)-3-methoxy-6H-[1]benzofuro[3a,3,2-ef][2]benzazepin-6-ol (Ib, Y₁=OH, Y₂=H, X=Br, Z₁= C₆H₁₂NO)

3,0 g (+)-BromNorgalanthamin (Ib Y₁=OH, Y₂=H, X=Br, Z₁=H), 4,8 g Kaliumcarbonat und 1,47 g N-(2-Chlorethyl)-morpholin Hydrochlorid werden in 30 ml Acetonitril bei Rückflusstemperatur gerührt. Nach 22 Stunden wird die Reaktionsmischung auf Raumtemperatur abgekühlt, das Lösungsmittel im Vakuum abdestilliert und der Rückstand mit 100 ml Wasser und 40 ml Ethylacetat versetzt. Nach dem Abtrennen wird die wässrige Phase mit 3x40 ml Ethylacetat extrahiert. Die vereinigten organischen Phasen werden über Natriumsulfat getrocknet, filtriert und unter Vakuum eingeengt. Das Produkt wird säulenchromatographisch (Laufmittel 96 % Ethanol) gereinigt.
Ausbeute: 1,9 g (48 % d. Th)
Smp 58-64°C
R_{f}: 0,2 (96 % Ethanol)
¹H-NMR (DMSO): δ 6.99 (s, 1H), 6.12 (d, 1H), 5.82 (dd, 1H), 4.55 (b, 1H), 4.37 (b, 1H), 4.20 (d, 1H), 4.05 (m, 2H), 3.79 (s, 3H), 3.51 (m, 4H), 3.32 (d, 1H), 3.28 (d, 1H), 2.99 (d, 1H), 2.51 (m, 2H), 2.35 (m, 4H), 2.25 (d, 1H), 2.00 (m, 2H), 1.49 (d, 1H);
¹³C-NMR (CDCl₃) : δ 146.9 (s), 144.6 (s), 134.9 (s), 129.6 (d), 128.7 (s), 127.4 (d), 116.3 (d), 113.3 (s), 87.8 (d), 67.1 (2t), 60.5 (d), 57.1 (2t), 56,7 (q), 55.9 (t), 54.5 (2t), 52.2 (t), 49.4 (t), 34.0 (t), 31.7 (t);

### Beispiel 59

### (4aS,8aS)-Δ^{5,6}-4a,5,9,10,11,12-Hexahydro-11-methyl-3-methoxy-6-phenyl-6H-[1]benzofuro[3a,3,2-ef][2]benzazepine (Ic, Y₃=Phenyl, X=H, Z₂= CH₃)

Zu einer Mischung von 1,16 g Magnesium in 15 ml Tetrahydrofuran werden 3,35 ml Brombenzol getropft. Das entstandene Reaktionsgemisch wird eine Stunde intensiv gerührt, mit einer Lösung von 3,0 g (-) Narwedin, hergestellt gemäß EP-A-0787115, in 50 ml Tetrahydrofuran versetzt und weiter gerührt. Nach 2 Stunden werden 60 ml Wasser und 40 ml 2N HCl zur Reaktionsmischung getropft und die entstandene Suspension wird bei 60°C gerührt. Nach 50 min wird die Reaktionsmischung auf Raumtemperatur abgekühlt, der pH-Wert mit Ammoniak-Lösung auf 9 eingestellt und mit 3x100 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=99:1) gereinigt.
Ausbeute: 1,8 g (49 % d. Th), farbloser Schaum
R_{f}: 0,35 (Chloroform:MeOH=99:1)
¹H-NMR (CDCl₃): δ 7.50 (d, 2H), 7.39 (m, 3H), 6.65 (b, 2H), 6.42 (d, 1H), 6.33 (m, 2H), 5.00 (d, 1H), 4.28 (d, 1H), 3.89 (s, 3H), 3.75 (d, 1H), 3.51 (t, 1H), 3.09 (d, 1H), 2.41 (s, 3H), 2.13 (dt, 1H), 1.79 (dd, 1H);
¹³C-NMR (CDCl₃): δ 148.2 (s), 144.3 (s), 139.8 (s), 139.5 (s), 131.6 (s), 131.2 (d), 130.0 (s), 129.0 (2d), 128.6 (d), 126.6 (2d), 123.2 (d), 122.2 (d), 116.1 (d), 110.7 (d), 86.5 (d), 60.8 (t), 56.2 (q), 54.5 (t), 48.7 (t), 42.2 (q), 35.4 (t);

### Beispiel 60

### (4aS,8aS)-Δ^{5,6}-4a, 5,9,10,11,12-Hexahydro-6,11-dimethyl-3-methoxy-6H-[1]benzofuro[3a,3,2-ef][2]benzazepine (Ic, Y₃=CH₃, X=H, Z₂=CH₃)

Eine Lösung von 2,04 g (-) Narwedin, hergestellt gemäß EP-A-0787115, in 60 ml Tetrahydrofuran wird mit 10,0 ml Methylmagnesiumbromid in Diethylether tropfenweise versetzt und bei Raumtemperatur gerührt. Nach 45 min werden 60 ml Wasser und 20 ml 2N HCl zur Reaktionsmischung getropft, und die entstandene Suspension wird bei 60 °C gerührt. Nach 50 min wird die Reaktionsmischung auf Raumtemperatur abgekühlt, der pH-Wert mit Ammoniak-Lösung auf 9 eingestellt und mit 3x100 ml Ethylacetat extrahiert. Die vereinigten organischen Phasen werden über Natriumsulfat getrocknet, filtriert und unter Vakuum eingeengt. Das Produkt wird säulenchromatographisch (Chloroform:MeOH=98:2) gereinigt.
Ausbeute: 2,18 g (72 % d. Th), farbloser Schaum
R_{f}: 0,5 (Chloroform:MeOH=99:1)
¹H-NMR (CDCl₃): δ 6.57 (m, 2H), 6.01 (d, 1H), 5.87 (d, 1H), 5.69 (m, 1H), 4.80 (d, 1H), 4.15 (d, 1H), 3.80 (s, 3H), 3.68 (d, 1H), 3.34 (t, 1H), 3.01 (d, 1H), 2.41 (s, 3H), 2.09 (dt, 1H), 1.91 (s, 3H), 1.71 (dd, 1H);
¹³C-NMR (CDCl₃): δ 148.2 (s), 144.2 (s), 136.6 (s), 131.8 (s), 130.2 (s), 130.0 (d), 125.2 (d), 121.9 (d), 115:3 (d), 110.5 (d), 88.9 (d), 60.8 (t), 56.2 (q), 54.6 (t), 48.5 (t), 42.4 (q), 35.4 (t), 22.2 (q) ;

### Beispiel 61

### (4aS,8aS)-Δ^{5,6}-4a,5,9,10,11,12-Hexahydro-6-(isopropyl)-11-methyl-3-methoxy-6H-[1]benzofuro[3a,3,2-ef][2]benzazepine (Ic, Y₃=Isopropyl, X=H, Z₂=CH₃)

Zu 220 mg Magnesiumspäne in 1.5 ml absolutem Tetrahydrofuran werden unter Stickstoffatmosphäre 0.66 ml 2-Brompropan zugetropft. 15 Minuten nach dem Start der Grignardreaktion wird unter Eiskühlung eine Lösung von 500 mg Narwedin, hergestellt gemäß EP-A-787115, in 12 ml absolutem Tetrahydrofuran zugetropft und bei Raumtemperatur gerührt. Nach 2 Stunden wird das Reaktionsgemisch unter Eis-Kühlung mit 30 ml Wasser hydrolysiert, mit 2 N Salzsäure angesäuert und bei 60°C 30 min gerührt. Anschließend wird die Lösung mit konzentrierter, wässriger Ammoniak-Lösung basisch eingestellt und dreimal mit je 30 ml Essigsäureethylester extrahiert. Die vereinigten organischen Phasen werden einmal mit gesättigter wässriger Natriumchloridlösung gewaschen, über Natriumsulfat getrocknet filtriert und eingedampft. Das Produkt wird säulenchromatographisch (Chloroform:MeOH=97:3) gereinigt.
Ausbeute: 399 mg (69 % d. Th), ölige Substanz
R_{f}: 0,55 (Chloroform:MeOH=97:3)
¹H-NMR (CDCl₃): δ 1,76-1,81 (m, 6H), 1,64 (ddd, 1H), 2,13 (ddd, 1H), 1,97 (ddd, 1H), 2,37 (ddd, 1H), 3,06 (ddd, 1H), 3,31 (ddd, 1H), 2,48 (s, 3H), 3,80 (s, 3H), 3,66 (d, 1H), 4,10 (d, 1H), 4,62 (b, 1H), 5,76 (d, 1H), 6,51 (d, 1H), 6,56 (d, 1H), 6,61 (d, 1H) ¹³C-NMR (CDCl₃) : δ 19,8 (q), -20,7 (q), 26,3 (t), 34,8 (t), 48,1 (s), 53,9 (t), 41,9 (q), 55,6 (q), 60,6 (t), 128,8 (s), 89,0 (d), 122,9 (d), 110,6 (d), 121,1 (d), 121,4 (s), 124,4 (d), 130,8 (s), 133,6 (s), 143,7 (s), 146,3 (s),
Zusammenfassend kann gesagt werden, dass sich die Derivate des 4a,5,9,10,11,12-Hexahydro-benzofuro[3a,3,2][2]benzazepin mit den allgemeinen Formeln Ia, Ib und Ic nicht nur in effizienter Weise mit der gewünschten optischen Reinheit im Industriemaßstab herstellen lassen, sondern dass diese aufgrund ihrer pharmakologischen Wirkung auch für die Herstellung von Arzneimitteln für die Behandlung verschiedenster Krankheitsbilder, insbesondere von Erkrankungen des zentralen Nervensystems (ZNS) geeignet sind.

## Patentansprüche

1. Verfahren zum Herstellen von Derivaten des 4a,5,9,10,11,12-Hexahydro-benzofuro [3a,3,2][2]benzazepin mit der allgemeinen Formel Ia bzw. Ib 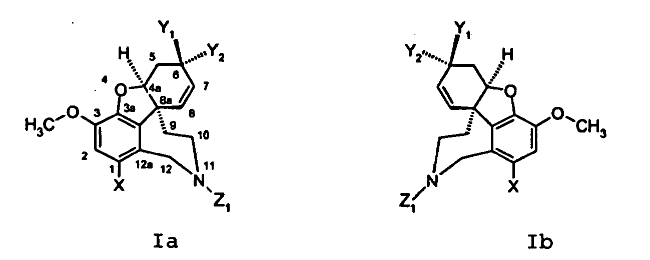 und dessen Salzen, wobei
• Ia optisch aktive (-) Derivate des Galanthamin und Ib optisch aktive (+)-Derivate des Galanthamin sind, die in zu einander spiegelbildlich räumlicher Anordnung vorliegen, und worin
• Y₁ und Y₂ wechselweise H oder OH,
• X = H oder Br sind und
• Z₁ = eine Gruppe mit folgender Formeldarstellung 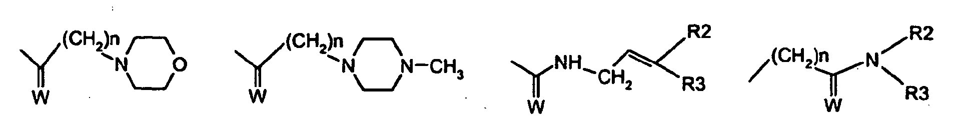 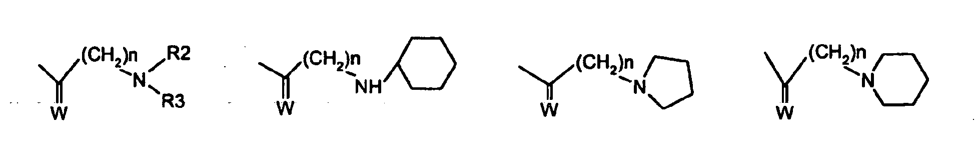 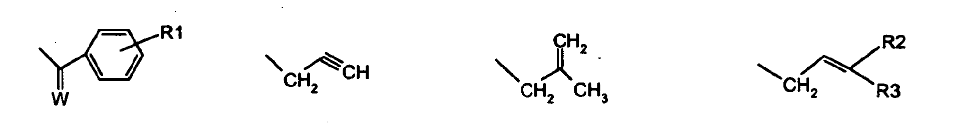 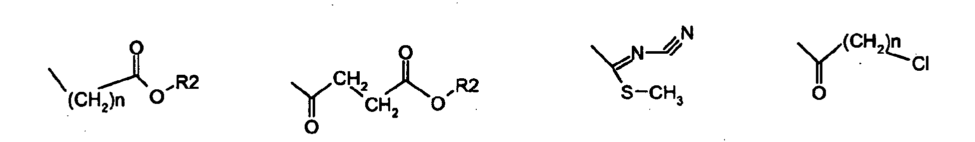 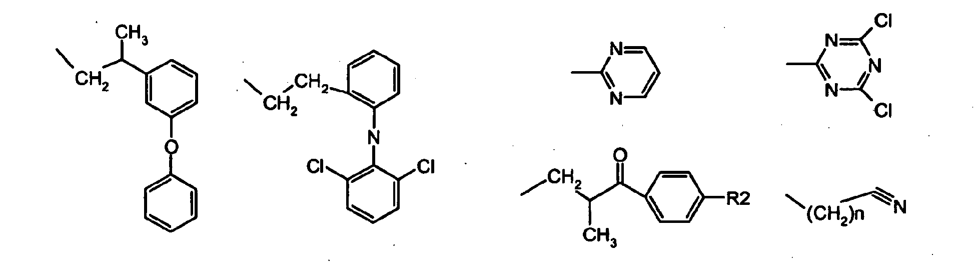
ist, worin
• R₁ = H, Cl, Br, I, F, OH, geradkettiges oder verzweigtes (C₁-C₆) alkyl, geradkettiges oder verzweigtes (C₁-C₆) alkyloxy, NO₂, NR₂R₃,
• R₂ = R₃ = H, geradkettiges oder verzweigtes (C₁-C₆) alkyl
• W = H, O, S
• n = 0, 1-6 sind,
**dadurch gekennzeichnet, dass** ein optisch aktives 11-Norgalanthamin-Derivat durch Behandlung mit verdünnter Säure in ein 6 Epi-Derivat des Galanthamin umgewandelt wird, dass bei der Säurebehandlung die räumliche Anordnung am Kohlenstoffatom 6 verändert wird, wogegen die räumliche Anordnung an den asymmetrischen Kohlenstoffatomen 4a und 8a unverändert bleibt und dass Alkylierungs- oder Acylierungsreaktionen in einem Lösungsmittel ausgewählt aus der Gruppe Toluol, Acetonitril, Ethanol, Aceton, 2-Butanon, Dimethylformamid oder Chloroform durchgeführt werden.

2. Verfahren nach Anspruch 1, **dadurch gekennzeichnet, dass** die Säurebehandlung mit verdünnter Salzsäure erfolgt.

## Claims

1. Method for the production of derivatives of 4a,5,9,10,11,12-hexahydro-benzofuro[3a,3,2][2]benzazepine with the general formula Ia or Ib 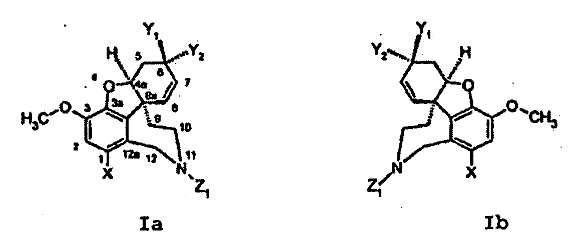 and the salts thereof,
• Ia being optically active (-) derivatives of galanthamine and Ib being optically active (+) derivatives of galanthamine, which are present in a spatial arrangement in mirror-image to each other, and in which
• Y₁ and Y₂ are alternately H or OH,
• X = H or Br and
• Z₁ = a group with the following formula representation 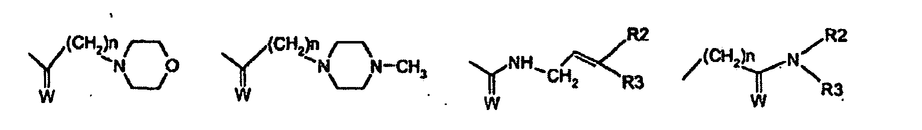 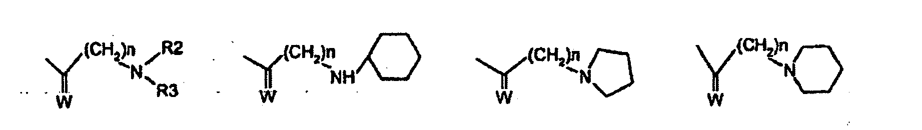 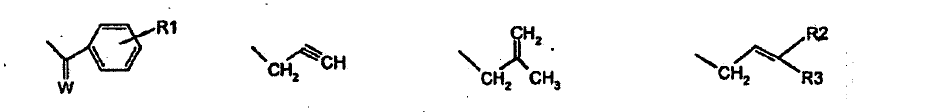 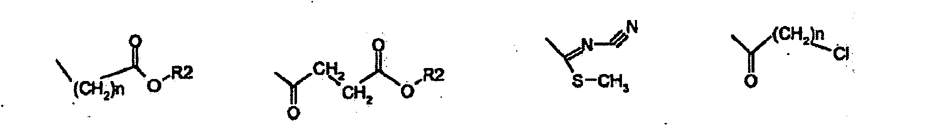 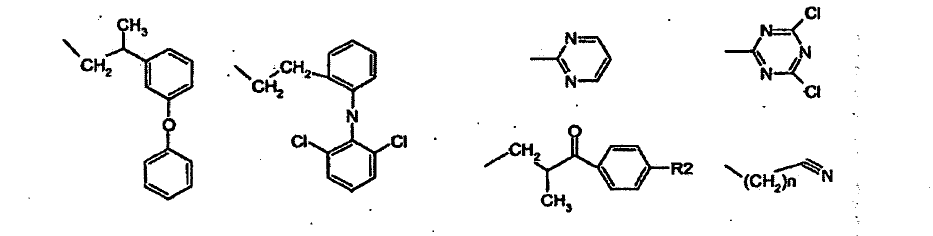
in which
• R₁ = H, Cl, Br, I, F, OH, straight-chain or branched (C₁-C₆) alkyl, straight-chain or branched (C₁-C₆) alkyloxy, NO₂, NR₂R₃,
• R₂ = R₃ = H, straight-chain or branched (C₁-C₆) alkyl
• W=H,O,S
• n = 0, 1-6,
**characterised in that** an optically active 11-norgalanthamine derivative is converted by treatment with diluted acid into a 6 epi-derivative of galanthamine, **in that** during the acid treatment the spatial arrangement at the carbon atom 6 is changed, whereas the spatial arrangement at the asymmetrical carbon atoms 4a and 8a remains unchanged and **in that** alkylation or acylation reactions are implemented in a solvent selected from the group toluene, acetonitrile, ethanol, acetone, 2-butanone, dimethylformamide or chloroform.

2. Method according to claim 1, **characterised in that** the acid treatment is effected with diluted hydrochloric acid.

## Revendications

1. Procédé de préparation de dérivés de 4a,5,9, 10,11,12-hexahydrobenzofuro [3a,3,2] [2] benzazépine répondant aux formules générales Ia respectivement Ib 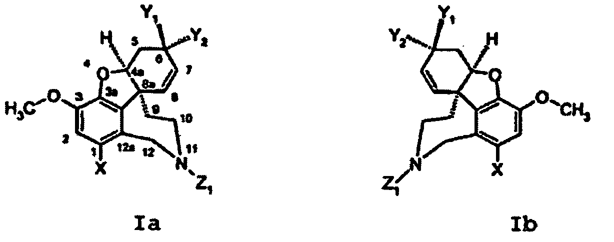 et leurs sels,
Ia étant des dérivés (-) optiquement actifs de la galanthamine et Ib étant des dérivés (+) optiquement actifs de la galanthamine, lesquels se présentent sous des arrangements dans l'espace images l'une de l'autre dans un miroir, et dans lesquelles
Y₁ et Y₂ sont alternativement H ou OH,
X = H ou Br et
Z₁ = un groupe de formule suivante 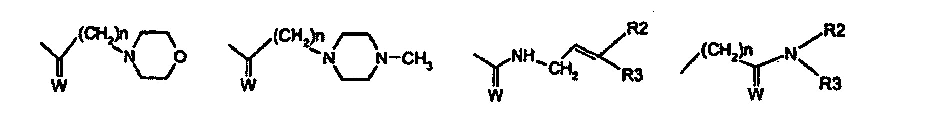 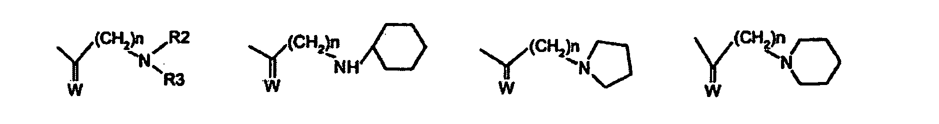 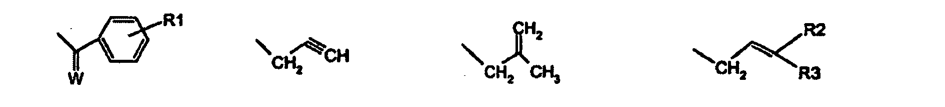 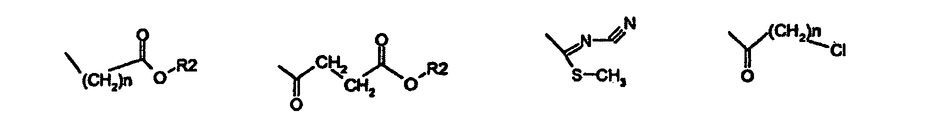 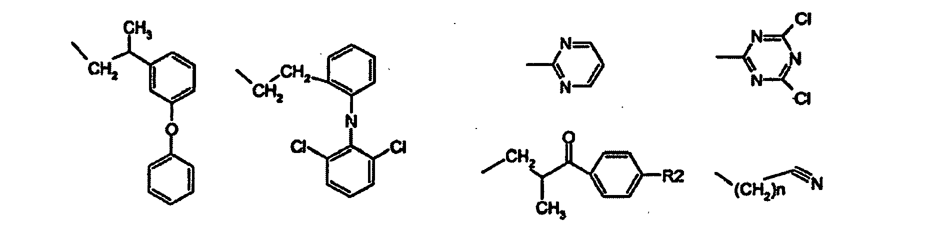 dans lesquelles,
R₁ = H, Cl, Br, I, F, OH, un groupe alkyle en C₁ à C₆ à chaîne droite ou ramifiée, un groupe alkyloxy en C₁ à C₆ à chaîne droite ou ramifiée, NO₂, NR₂R₃,
R₂ = R₃ = H, un groupe alkyle en C₁ à C₆ à chaîne droite ou ramifiée
W = H, O, S
n = 0, 1 à 6,
**caractérisé en ce qu'**un dérivé de 11-norgalanthamine optiquement actif est converti en un dérivé 6 épis de la galanthamine par traitement avec de l'acide dilué, lors du traitement acide, l'agencement spatial est modifié au niveau de l'atome de carbone 6, tandis que l'agencement spatial au niveau des atomes de carbone asymétriques 4a et 8a reste inchangé et **en ce que** des réactions d'alkylation ou d'acylation sont effectuées dans un solvant choisi dans le groupe constitué du toluène, de l'acétonitrile, de l'éthanol, de l'acétone, de la 2-butanone, du diméthylformamide ou du chloroforme.

2. Procédé selon la revendication 1, **caractérisé en ce que** le traitement acide est effectué avec de l'acide chlorhydrique dilué.
